(19) Europäisches Patentamt
European Patent Office
Office européen des brevets

(11) EP 3 533 322 A1

(12) EUROPEAN PATENT APPLICATION
published in accordance with Art. 153(4) EPC

(43) Date of publication:
**04.09.2019 Bulletin 2019/36**

(21) Application number: **17858412.4**

(22) Date of filing: **03.10.2017**

(51) Int Cl.:
***A01H 5/06*** *(2018.01)*
***A23L 5/43*** *(2016.01)*
***C12N 15/09*** *(2006.01)*
***C12Q 1/68*** *(2018.01)*
***A01H 1/02*** *(2006.01)*

(86) International application number:
**PCT/JP2017/036028**

(87) International publication number:
**WO 2018/066575 (12.04.2018 Gazette 2018/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.10.2016 JP 2016197640**

(71) Applicant: **San-Ei Gen F.F.I., INC.**
**Toyonaka-shi, Osaka 561-8588 (JP)**

(72) Inventors:
- **SAKAI, Tetsufumi**
  **Chikugo-shi**
  **Fukuoka 833-0041 (JP)**
- **YOSHINAGA, Masaru**
  **Kasai-gun**
  **Hokkaido 082-0081 (JP)**
- **TAKAHATA, Yasuhiro**
  **Koshi-shi**
  **Kumamoto 861-1192 (JP)**
- **KAI, Yumi**
  **Miyakonojo-shi**
  **Miyazaki 885-0091 (JP)**
- **KOBAYASHI, Akira**
  **Miyakonojo-shi**
  **Miyazaki 885-0091 (JP)**
- **KATAYAMA, Kenji**
  **Tsukuba-shi**
  **Ibaraki 305-8518 (JP)**
- **FUJITA, Toshiro**
  **Kurume-shi**
  **Fukuoka 839-8503 (JP)**
- **ONISHI, Hironori**
  **Toyonaka-shi**
  **Osaka 561-8588 (JP)**
- **MORIYUKI, Kazuya**
  **Toyonaka-shi**
  **Osaka 561-8588 (JP)**
- **NISHIYAMA, Koji**
  **Toyonaka-shi**
  **Osaka 561-8588 (JP)**

(74) Representative: **Vossius & Partner**
**Patentanwälte Rechtsanwälte mbB**
**Siebertstrasse 3**
**81675 München (DE)**

# (54) COLORED SWEET POTATO HAVING HIGH ANTHOCYANIN CONTENT, PROCESSED PRODUCT THEREOF, AND METHOD FOR DETERMINING VARIETY THEREOF

(57) The present invention provides a colored sweet potato that has a high anthocyanin content, and that is useful as a pigment material. Further, the present invention also provides a processed product of the colored sweet potato useful as a purple pigment, more specifically, an extract composition, as well as the purified matter thereof (including roughly purified matter).

A colored sweet potato having the following characteristics is used.
(A) the color value (530 nm) per gram of wet weight of colored sweet potato (color value (530nm)/g) is not less than 15;
(B) the absorbance ratio (320 nm/530 nm) per gram of wet weight of colored sweet potato (absorbance ratio (320 nm/530 nm)/g) is not less than 1.5;
(C) color value (530nm)/g × absorbance ratio (320nm/530nm)/g = not less than 30; and
(D) LTR retrotransposon (Rtsp-1) is inserted into at least two positions of the genome sequence, and an amplified product having a fragment length of 500 to 530bp is produced when a nucleic acid amplification reaction is performed using, as a test material, a part of a plant, and at least one of primer set 1 containing a forward primer having the base sequence of SEQ ID NO:1 and a reverse

EP 3 533 322 A1

primer having the base sequence of SEQ ID NO:2, and primer set 2 containing a forward primer having the base sequence of SEQ ID NO:1 and a reverse primer having the base sequence of SEQ ID NO:3.

Sequence Listing

SEQUENCE LISTING

<110> San-Ei Gen F.F.I.,Inc.

<120> Colored sweet potato having high anthocyanin content, processed product thereof, and method for determining variety thereof

<130> AC1568 EP S3

<140> EP 17 85 8412.4
<141> 2017-10-03

<150> JP 2016-197640
<151> 2016-10-05

<160> 10

<170> PatentIn version 3.5

<210> 1
<211> 27
<212> DNA
<213> Artificial Sequence

<220>
<223> Forward Primer

<400> 1
atctaatctt caagtgggag attgtcg 27

<210> 2
<211> 50
<212> DNA
<213> Artificial Sequence

<220>
<223> Reverse Primer

<400> 2
ggtccaatgc aagtaaggta tacaacttaa acctcttatg tctatgaagt 50

<210> 3
<211> 50
<212> DNA
<213> Artificial Sequence

<220>
<223> Reverse Primer

<400> 3
gaaacacttg atgtgaactc cacaacatga tgagaattac ttgtggcaac 50

<210> 4
<211> 26
<212> DNA
<213> Artificial Sequence

<220>
<223> Reverse Primer

<400> 4

gaagttgccc aaacaatgca atcagc 26

<210> 5
<211> 26
<212> DNA
<213> Artificial Sequence

<220>
<223> Reverse Primer

<400> 5
cacaatgcct tcattgtctt gaaccc 26

<210> 6
<211> 23
<212> DNA
<213> Artificial Sequence

<220>
<223> Reverse Primer

<400> 6
gttggctgct caacctcagt agc 23

<210> 7
<211> 23
<212> DNA
<213> Artificial Sequence

<220>
<223> Reverse Primer

<400> 7
ccaatgtgcg aaggcactac tcc 23

<210> 8
<211> 22
<212> DNA
<213> Artificial Sequence

<220>
<223> Reverse Primer

<400> 8
ggaccaatgc tgggacaagg tc 22

<210> 9
<211> 26
<212> DNA
<213> Artificial Sequence

<220>
<223> Reverse Primer

<400> 9
cctcgagctg ctaaagtact tattgg 26

<210> 10
<211> 21
<212> DNA
<213> Artificial Sequence

<220>

<223> Reverse Primer

<400> 10
catttccacc gtccaggagc c 21

## Description

### Technical Field

The present invention relates to a colored sweet potato that has a high anthocyanin content, and that is useful as a pigment material. The present invention also relates to a processed product of the colored sweet potato, more specifically, an extract composition, as well as the purified matter thereof (including roughly purified matter). The present invention further relates to use of the processed product as, for example, a purple pigment. Further, the present invention also relates to a method for determining a variety of colored sweet potato, and a reagent used in the method.

### Background Art

Colored sweet potato (also referred to as red sweet potato, or purple sweet potato) contains a large amount of anthocyanin. Previously, colored sweet potato with an increased anthocyanin content, such as *Akemurasaki, Ayamurasaki, Murasakimasari,* etc., was developed as an improved variety; and widely commercially distributed (NPL 1) and used in various processed foods. In particular, the anthocyanin derived from colored sweet potatoes is superior in terms of heat resistance, light resistance, and vividness in color tone, compared with the anthocyanin derived from red cabbage, grapes, purple corn, red radish, and the like; therefore, it is more frequently used in the food processing field or the like as a red-to-purple edible pigment. Further, there are reports of excellent functions of anthocyanin, including antioxidation activity, liver function improving activity, blood pressure elevation inhibitory effects, antimutagenic effects, angiotensin I-converting enzyme inhibitory activity, $\alpha$ glucosidase inhibitory activity, bovine early embryo generation ratio decrease inhibitory effects, blood glucose level increase inhibitory effects, vasorelaxant effects, blood flow increasing effects, and the like. Therefore, the use of the anthocyanin for functional foods has been expected.

In sweet potato (*Kansho*), LTR retrotransposon Rtsp-1 having metastasis activity has previously been identified (NPL 2), and it has been confirmed that the variety of sweet potato can be specified by using a DNA marker utilizing the insertional polymorphism thereof. Recently, sequence information of the Rtsp-1 insertion site has been reported with regard to typical varieties of colored sweet potato, as well as 38 varieties/breeding lines around their parental strains, by exhaustive analysis using a next-generation sequencer (NPL 3).

### Citation List

#### Non-Patent Literature

NPL 1: NARO List of Providers of Varieties Grown in Kyushu Okinawa Agricultural Research Center (2016.4.18 edition) (searched on August 6, 2016), internet <URL: http: //www.naro.affrc.go.jp/karc/contents/files/seed imo.pdf> NPL 2: Tahara, M., et al., Mol Gen. Genomics 272: (2004) 116-127 NPL 3: Monden, Y., et al., DNA Res. 21 (2014), 491-498

### Summary of Invention

#### Technical Problem

An object of the present invention is to provide a new variety of colored sweet potato that has a high pigment (anthocyanin) content, and that is suitable for a pigment material. Further, another object of the present invention is to provide a processed product of the colored sweet potato, more specifically, an extract composition, as well as the purified matter thereof (including roughly purified matter). A further object of the present invention is to provide use of these processed products for, for example, a purple pigment.

A still further object of the present invention is to provide a method for determining a variety of edible colored sweet potato, particularly a variety of colored sweet potato contained in an edible composition, as well as a reagent and a reagent kit used for the determination.

#### Solution to Problem

The inventors of the present invention carried out extensive research in view of the above objects in order to produce a new colored sweet potato, and succeeded in obtaining the target colored sweet potato that has a high purple pigment (anthocyanin) content, which is suitable for a pigment material, by hybridization of a mother variety (*Kyukei* 04208-2) and a father variety (*Kyukei* 04222-50), and screening. This colored sweet potato not only has a pigment content higher than that of existing colored sweet potato, but also has a high content of polyphenol having an antioxidant effect. Therefore, the colored sweet potato is characterized by containing a pigment in a stable state. Further, since this sweet potato has an LTR retrotransposon (Rtsp-1) at a site different from that of existing edible colored sweet potato, it was confirmed that this colored sweet potato is a new variety. As described above, since the new variety of colored sweet potato has a characteristic gene sequence that is different from the gene sequence of existing edible colored sweet potato, it becomes possible to determine whether a colored sweet potato or a colored sweet potato contained in an edible composition corresponds to the variety of the present invention by using the gene sequence as an index.

The present invention was completed based on the above findings, and has the following embodiments.

(I) New Colored Sweet Potato

(I-1) A colored sweet potato having at least one of characteristics (D) and (E), in addition to the following characteristics (A) to (C):

(A) the color value (530 nm) per gram of wet weight of colored sweet potato (color value (530nm)/g) is not less than 15;
(B) the absorbance ratio (320 nm/530 nm) per gram of wet weight of colored sweet potato (absorbance ratio (320 nm/530 nm)/g) is not less than 1.5;
(C) color value (530nm)/g $\times$ absorbance ratio (320nm/530nm)/g = not less than 30;
(D) LTR retrotransposon (Rtsp-1) is inserted into at least two positions of the genome sequence, and an amplified product having a fragment length of 500 to 530bp is produced when a nucleic acid amplification reaction is performed using, as a test material, a part of a plant, and at least one of primer set 1 containing a forward primer having the base sequence of SEQ ID NO:1 and a reverse primer having the base sequence of SEQ ID NO:2, and primer set 2 containing a forward primer having the base sequence of SEQ ID NO:1 and a reverse primer having the base sequence of SEQ ID NO:3; and
(E) the total amount of aroma components per color value (530 nm) is not more than 40%, based on the total amount of aroma components per color value (530 nm) of the existing variety *Akemurasaki.*

(1-2) The colored sweet potato according to (1-1), wherein the aroma components comprise 23 kinds of aroma components constituted of butanol, isoamyl alcohol, acetoin, acetol, nonanal, acetic acid, 2-ethylhexanol, benzaldehyde, isobutyric acid, $\gamma$-butyrolactone, menthol, $\alpha$-terpineol, guaiacol, phenethyl alcohol, 3,7-dimethyloct-1-en-3,7-diol, 3-hydroxy-2-pyrone, pantolactone, cis-1,8-terpin, 4-vinylguaiacol, 5-(hydroxymethyl)furfural, vanillin, zingerone, and vanillyl alcohol.
(1-3) The colored sweet potato according to (1-1) or (I-2), wherein the colored sweet potato is derived from a mother strain (*Kyukei* 04208-2) and a father strain (*Kyukei* 04222-50), and the mother strain is a sweet potato having the same characteristic as that defined in (D) of (1-1).
(1-4) The colored sweet potato according to any one of (1-1) to (1-3), wherein the colored sweet potato is a colored sweet potato for use in a pigment material.

(II) Processed Product of Colored Sweet Potato, and Pigment Composition

(II-1) An extract composition of the colored sweet potato according to any one of (I-1) to (1-4) or a purified matter thereof.
(II-2) The extract composition or the purified matter thereof according to (II-1), which is a pigment composition.
(II-3) A pigment composition derived from a colored sweet potato containing at least anthocyanin pigments of pigment YGM-0e, pigment YGM-4b, pigment YGM-5a, pigment YGM-6, and pigment YGM-2, and the content ratio of each pigment satisfies at least one of (1) to (4), as peak area ratio detected by HPLC under the following conditions:

(1) a/b:0.3 to 2
(2) c/b:2.3 to 10
(3) c/d:1.2 to 5
(4) e/a:0.1 to 4.5

a: peak area of pigment YGM-0e
b: peak area of pigment YGM-4b
c: peak area of pigment YGM-5a

d: peak area of pigment YGM-6
e: peak area of pigment YGM-2

Conditions of HPLC Analysis

ODS reverse-phase column (linking group: triacontyl group): pore size (14 nm), specific surface area (300 $m^2/g$), pore volume (1.05 mg/mL), diameter and length ($\phi$4.6×250 nm)
Column temperature: 40°C
Mobile phase: (a) 1v/v% formic acid aqueous solution, (b) acetonitrile

Gradient conditions:

0→15 minutes, (a) 95%→82%, (b) 5%→18%
15→45 minutes, (a) 82%→30%, (b) 18%→70%
45→55 minutes, (a) 30%→20%, (b) 70%→80%
55→60 minutes, (a) 20%→0%, (b) 80%→100%
Flow Rate: 1.0 mL/min
Sample injection amount: 20 $\mu$L
Detection: Photodiode array detector (530 nm).
(II-4) The pigment composition according to (II-3), wherein the peak area ratio is calculated from the following a to e obtained by measurement using the following HPLC apparatus and column:
HPLC apparatus: JASCO LC-2000Plus series (JASCO Corporation) Column: Develosil C30-UG-5($\phi$4.6×250nm) (Nomura Chemical Co., Ltd.)

a: peak area of pigment YGM-0e detected at a retention time of about 17.52 minutes;
b: peak area of pigment YGM-4b detected at a retention time of about 22.07 minutes;
c: peak area of pigment YGM-5a detected at a retention time of about 22.23 minutes;
d: peak area of pigment YGM-6 detected at a retention time of about 22.84 minutes; and
e: peak area of pigment YGM-2 detected at a retention time of about 20.61 minutes.

(II-5) The pigment composition according to (II-3) or (II-4), wherein the total amount of aroma components is not more than 120 ppm when the color value at the maximum absorption wavelength around a wavelength of 530 nm is $E^{10\%}_{lcm}$=80.
(1-6) The pigment composition according to any one of (II-3) to (II-5), wherein the colored sweet potato is the colored sweet potato according to any one of (I-1) to (1-4).

(III) Methods for Determining Variety of Sweet Potato, and Reagent Used for the Method

(III-1) A method for determining a variety of an edible colored sweet potato, or an edible colored sweet potato used as a raw material of an edible composition comprising, as a raw material, an edible colored sweet potato, the method comprising the steps (1) and (2), or the steps (1') and (2'):

(1) a step of performing a nucleic acid amplification reaction using, as a template, DNA prepared from an edible colored sweet potato or an edible composition containing an edible colored sweet potato, and at least one of primer set 1 containing a forward primer having the base sequence of SEQ ID NO:1 and a reverse primer having the base sequence of SEQ ID NO:2, and primer set 2 containing a forward primer having the base sequence of SEQ ID NO:1 and a reverse primer having the base sequence of SEQ ID NO:3; and
(2) a step of confirming the presence or absence of production of amplified product having a fragment length of 500 to 530bp by the nucleic acid amplification reaction; or

(1') a step of performing a nucleic acid amplification reaction using, as a template, DNA prepared from an edible colored sweet potato or an edible composition containing an edible colored sweet potato as a raw material, and at least one of primer sets 3 to 9 containing a forward primer having the base sequence of SEQ ID NO:1 and a reverse primer having the base sequence of any one of SEQ ID NOs:4 to 10; and

(2') a step of confirming the presence or absence of production of amplified product having a fragment length of 550 to 650bp by the nucleic acid amplification reaction.

(III-2) The method for determining a variety of an edible colored sweet potato according to (III-1), further comprising the following step (3):
(3) a step of determining, when production of corresponding amplified product is confirmed, that the edible colored sweet potato or the edible colored sweet potato contained in the edible composition is the colored sweet potato according to any one of (I-1) to (1-4).
(III-3) The method according to (III-2), wherein the edible colored sweet potato is *Kyushu* No.180.
(III-4) A reagent for determining a variety of a colored sweet potato, comprising at least one primer set selected from the group consisting of the following (a) to (i):

(a) primer set 1 containing a forward primer having the base sequence of SEQ ID NO:1 and a reverse primer having the base sequence of SEQ ID NO:2,
(b) primer set 2 containing a forward primer having the base sequence of SEQ ID NO:1 and a reverse primer having the base sequence of SEQ ID NO:3,
(c) primer set 3 containing a forward primer having the base sequence of SEQ ID NO:1 and a reverse primer having the base sequence of SEQ ID NO:4,
(d) primer set 4 containing a forward primer having the base sequence of SEQ ID NO:1 and a reverse primer having the base sequence of SEQ ID NO:5,
(e) primer set 5 containing a forward primer having the base sequence of SEQ ID NO:1 and a reverse primer having the base sequence of SEQ ID NO:6,
(f) primer set 6 containing a forward primer having the base sequence of SEQ ID NO:1 and a reverse primer having the base sequence of SEQ ID NO:7,
(g) primer set 7 containing a forward primer having the base sequence of SEQ ID NO:1 and a reverse primer having the base sequence of SEQ ID NO:8,
(h) primer set 8 containing a forward primer having the base sequence of SEQ ID NO:1 and a reverse primer having the base sequence of SEQ ID NO:9, and
(i) primer set 9 containing a forward primer having the base sequence of SEQ ID NO:1 and a reverse primer having the base sequence of SEQ ID NO:10.

(III-5) The reagent for determining a variety of a colored sweet potato according to (III-4), for use in the execution of the method for determining a variety of an edible colored sweet potato according to any one of (III-1) to (III-3).
(III-6) A kit used to determine a variety of a colored sweet potato, the kit comprising the reagent for determining a variety of a colored sweet potato according to (III-4) or (III-5) .
(III-7) The kit for determining a variety of a colored sweet potato according to (III-6), for use in the execution of the method for determining a variety of an edible colored sweet potato according to any one of (III-1) to (III-3).

Advantageous Effects of Invention

The colored sweet potato of the present invention has, compared with previously known colored sweet potatoes, such as *Akemurasaki, Ayamurasaki,* and *Murasakimasari,* a higher pigment (anthocyanin) content and a higher color value, as well as a higher content of chlorogenic acid having an antioxidant effects for stabilizing the pigment; and is thus useful as a purple sweet potato as a pigment material for preparing a pigment. Further, the pigment composition prepared from the colored sweet potato (colored sweet potato pigment) has a low content of aroma component that may have an influence when it is added to a food or the like, and also has desirable stability with respect to light and heat (pigment residual ratio: high, color tone change: small).
Further, the reagent and the kit for use in determination of the variety of colored sweet potato of the present invention is capable of determining the variety of the colored sweet potato of the present invention, while distinguishing it from previously known colored sweet potatoes. Therefore, the present invention makes it possible to determine, regarding an edible composition containing colored sweet potato, whether the colored sweet potato corresponds to the variety of the colored sweet potato of the present invention in a simple and highly accurate manner.

Brief Description of Drawings

Fig. 1: A schematic diagram of retrotransposon Rtsp-1, and a drawing showing the relative position with respect to forward primer (ppt primer) and reverse primer (CL1836 primer, CL1056 primer) used for genetic amplification of a genomic DNA sequence into which Rtsp-1 is inserted.

Fig. 2: A drawing showing the results of GC/MS analysis of the aroma components contained in a pigment extract of the new variety (*Kyushu* No.180) obtained in Example 2, together with the analysis results of the aroma components contained in the existing varieties (*Ayamurasaki, Murasakimasari, Akemurasaki*) (Example 3).

Fig. 3, lower left: A graph showing the relative amounts of the total amount of 23 kinds of aroma components contained in the pigment extracts of the existing varieties (*Ayamurasaki, Murasakimasari, Akemurasaki*), based on the total amount (=100) of the 23 kinds of aroma components contained in the pigment extract of the new variety (*Kyushu* No.180). Fig. 3, lower right: A graph showing the results of conversion of the total amount of the 23 kinds of aroma components to values per color value (530 nm).

Fig. 4: A drawing showing the results of analysis of the principal components of the pigment extract of the new variety (*Kyushu* No.180) obtained in Example 2, together with the results of the existing varieties (*Ayamurasaki, Murasakimasari, Akemurasaki*) (Example 3).

Fig. 5: A table showing HPLC profile of the pigment extracts of the varieties (*Ayamurasaki, Murasakimasari, Akemurasaki, Kyushu* No.180) prepared in Example 2 detected at a wavelength of 530 nm. Fig. 5A shows peak retention time (min) and peak area detected in the respective varieties, and Fig. 5B shows relative ratio (%) of each peak area based on the total peak area (= 100%) (Example 4-1).

Fig. 6: A graph showing a comparison between the respective varieties (*Ayamurasaki, Murasakimasari, Akemurasaki, Kyushu* No.180) in terms of peak areas (relative ratio %) of p-hydroxybenzoylated (cyanidin 3-sophoroside-5-glucoside) (YGM-Oc) and p-hydroxybenzoylated (peonidin 3-sophoroside-5-glucoside) (YGM-Oe) (Example 4-1).

Fig. 7A: A graph showing a comparison between the respective varieties (*Ayamurasaki, Murasakimasari, Akemurasaki, Kyushu* No.180) in terms of content ratios (relative ratio) of the pigment components (a:YGM-0e, b:YGM-4b, c:YGM-5a, d:YGM-6, e:YGM-2). Fig. 7A shows the results of a comparison between the respective varieties in terms of a/b (YGM-0e/YGM-4b) (Example 4-1) .

Fig. 7B: A graph showing a comparison between the respective varieties (*Ayamurasaki, Murasakimasari, Akemurasaki, Kyushu* No.180) in terms of content ratios (relative ratio) of the pigment components (a:YGM-0e, b:YGM-4b, c:YGM-5a, d:YGM-6, e:YGM-2). Fig. 7B shows the results of a comparison between the respective varieties in terms of c/b (YGM-5a/YGM-4b) (Example 4-1).

Fig. 7C: A graph showing a comparison between the respective varieties (*Ayamurasaki, Murasakimasari, Akemurasaki, Kyushu* No.180) in terms of content ratios (relative ratio) of the pigment components (a:YGM-0e, b:YGM-4b, c:YGM-5a, d:YGM-6, e:YGM-2). Fig. 7C shows the results of a comparison between the respective varieties in terms of c/d (YGM-5a/YGM-6) (Example 4-1) .

Fig. 7D: A graph showing a comparison between the respective varieties (*Ayamurasaki, Murasakimasari, Akemurasaki, Kyushu* No.180) in terms of content ratios (relative ratio) of the pigment components (a:YGM-0e, b:YGM-4b, c:YGM-5a, d:YGM-6, e:YGM-2). Fig. 7D shows the results of a comparison between the respective varieties in terms of e/a (YGM-2/YGM-0e) (Example 4-1) .

Fig. 8: Tables showing HPLC profile of ultraviolet component detected at a wavelength of 320 nm with regard to the pigment extracts of the varieties (*Ayamurasaki, Murasakimasari, Akemurasaki, Kyushu* No.180) prepared in Example 2. Fig. 8A shows peak retention time (min) and peak area detected in the respective varieties, and Fig. 8B shows values obtained by dividing each peak area by the total peak area detected at a wavelength of 530 nm (Example 4-1).

Fig. 9: A graph showing a comparison between the respective varieties in terms of the values obtained by dividing the peaks detected at a retention time of about 10.92 minutes and about 24.44 minutes, which are the peaks of ultraviolet component confirmed most in the new variety (*Kyushu* No.180) among the respective varieties; and the peak area detected at a retention time of about 24.69 minutes, which is the peak of ultraviolet component confirmed least in the new variety (*Kyushu* No.180) by the total peak area detected at a wavelength of 530 nm (Example 4-1) .

Fig. 10: A drawing showing the varieties (27 kinds) of sweet potato used for PCR amplification of the Rtsp-1 insertion site using ppt primer (SEQ ID NO:1) and CL1836 primer (SEQ ID NO:2), and the results thereof (Example 5).

Fig. 11: A drawing showing the varieties (27 kinds) of sweet potato used for PCR amplification of the Rtsp-1 insertion site using ppt primer (SEQ ID NO:1) and CL1056 primer (SEQ ID NO:3), and the results thereof (Example 5).

Fig. 12: A drawing showing a comparison between various varieties (*Ayamurasaki, Akemurasaki,* new variety (*Kyushu* No.180)) in terms of GC/MS analysis results (chromatogram) of colored sweet potato pigments prepared and purified in Example 6.

Fig. 13: Tables showing HPLC profile of the pigment compositions of the varieties (*Ayamurasaki, Akemurasaki, Kyushu* No.180) prepared and purified in Example 6 detected at a wavelength of 530 nm. Fig. 13A shows peak retention time (min) and peak area detected in the respective varieties, and Fig. 13B shows relative ratio (%) of each peak area based on the total peak area (= 100%) (Example 8).

Fig. 14: Graphs showing a comparison between the varieties (*Ayamurasaki, Akemurasaki, Kyushu* No.180) prepared and purified in Example 6 in terms of content ratios (relative ratio) of the pigment components (a:YGM-0e, b:YGM-4b, c:YGM-5a, d:YGM-6, e:YGM-2). The graphs show a comparison between those varieties in terms of a/b (YGM-0e/YGM-4b) (14A), c/b (YGM-5a/YGM-4b) (14B), c/d (YGM-5a/YGM-6) (14C), and e/a (YGM-2/YGM-0e) (14D) (Example 8).

Fig. 15: Tables showing HPLC profile of ultraviolet component detected at a wavelength of 320 nm with respect to the pigment compositions of the respective varieties (*Ayamurasaki, Akemurasaki, Kyushu* No.180). Fig. 15A shows peak retention time (min) and peak area detected in the respective varieties, and Fig. 15B shows values obtained by dividing each peak area by the total peak area detected at a wavelength of 530 nm (Example 8).

Fig. 16: A graph showing a comparison between the various varieties in terms of the values obtained by dividing the peak areas detected at a retention time of about 10.92 minutes, about 24.44 minutes, and about 24.69 minutes, which are the peaks of ultraviolet component confirmed most in the new variety (*Kyushu* No.180) among the respective varieties, by the total peak area detected at a wavelength of 530 nm (Example 8).

Fig. 17: Tables showing HPLC profile detected at a wavelength of 530 nm with regard to pigment compositions (pigment liquid extracts) prepared from colored sweet potato raised and harvested by vegetative propagation using the new variety (*Kyushu* No.180) produced in Example 1 as the seed potato, and having the same gene. Fig. 17A shows peak retention time (min) and peak area detected in the new variety, and Fig. 17B shows relative ratios (%) of the respective peak areas based on the total peak area (= 100%) (Example 10).

Fig. 18: Tables showing HPLC profile detected at a wavelength of 530 nm with regard to a concentrated liquid extract prepared from the colored sweet potato mentioned above. Fig. 18A shows peak retention time (min) and peak area detected in the new variety, and Fig. 18B shows relative ratios (%) of the respective peak areas based on the total peak area (= 100%) (Example 10).

Fig. 19A: An electrophoresis image of an amplified product obtained by PCR amplification using primer set 3 (Example 11). In the image, A to F lanes correspond to the results of *Kyushu* No.180, *Kyukei* 04222-50, *Kyukei* 04208-2, *Akemurasaki, Murasakimasari,* and *Ayamurasaki,* respectively, in this order (this is also applied to Figs. 19B to 19G below).

Fig. 19B: An electrophoresis image of an amplified product obtained by PCR amplification using primer set 4 (Example 11).

Fig. 19C: An electrophoresis image of an amplified product obtained by PCR amplification using primer set 5 (Example 11).

Fig. 19D: An electrophoresis image of an amplified product obtained by PCR amplification using primer set 6 (Example 11).

Fig. 19E: An electrophoresis image of an amplified product obtained by PCR amplification using primer set 7 (Example 11).

Fig. 19F: An electrophoresis image of an amplified product obtained by PCR amplification using primer set 8 (Example 11).

Fig. 19G: An electrophoresis image of an amplified product obtained by PCR amplification using primer set 9 (Example 11).

Description of Embodiments

(I) Colored Sweet Potato

The colored sweet potato of the present invention is characterized by having at least one of characteristics (D) and (E), in addition to characteristics (A) to (C), which are detailed below.

(A) The color value (530 nm) per gram of wet weight of colored sweet potato (color value (530nm)/g) is not less than 15.

(B) The absorbance ratio (320nm/530nm) per gram of wet weight of colored sweet potato (absorbance ratio (320nm/530nm)/g) is not less than 1.5.

(C) The value obtained by multiplying "color value (530nm)/g" by "absorbance ratio (320nm/530nm)/g" (color value (530nm)/g × absorbance ratio (320nm/530nm)/g) is not less than 30%.

(D) LTR retrotransposon (Rtsp-1) is inserted into at least two positions of the genome sequence, and an amplified product having a fragment length of 500 to 530bp is produced when a nucleic acid amplification reaction is performed using, as a test material, a part of a plant, and at least one of primer set 1 containing a forward primer comprising the base sequence of SEQ ID NO:1 and a reverse primer comprising the base sequence of SEQ ID NO:2, and primer set 2 containing a forward primer comprising the base sequence of SEQ ID NO:1 and a reverse primer comprising the base sequence of SEQ ID NO:3.

(E) The total amount of the aroma components per color value (530 nm) is not more than 40%, based on the total amount of the aroma components per color value (530 nm) of the existing variety *Akemurasaki.*

Sweet potato (*Satsuma-imo,* scientific name: *Ipomoea batatas,* also known as *Kansho, Karaimo,* or *Ryukyu-imo*) is a plant of genus *Ipomoea* in the family Convolvulaceae. In the present invention, "sweet potato" means a tuberous root (i.e., the edible portion) of a plant (preferably *Kansho: Ipomoea batatas* (L.) Lam) of genus *Ipomoea* in the family Convolvulaceae (*Ipomoea* in Convolvulaceae). Further, in the present invention, "colored sweet potato" means a sweet potato with a purple body (internal tuberous root) color.

The aforementioned characteristics of the colored sweet potato of the present invention are described below.

(A) The color value (530 nm) per gram of wet weight of the colored sweet potato is not less than 15.

This means that the colored sweet potato of the present invention has a deep purple color; more specifically, the colored sweet potato of the present invention contains a large amount of a purple pigment component.

In the present invention, "the color value (530 nm) per gram of wet weight of colored sweet potato" (may also be referred to as "color value (530nm)/g" in the present invention) is measured according to the "color value measurement method" disclosed in the Japanese Standards of Food Additives (8th Edition: Japan Ministry of Health, Labour and Welfare).

The "color value measurement method" of the Japanese Standards of Food Additives defines the color value as being expressed by a value ($E^{10\%}_{lcm}$) obtained by converting an absorbance of a colorant solution measured at the maximum absorption wavelength in the visible range to an absorbance of a 10 w/v% solution. For a colored sweet potato having a purple color, the maximum absorption wavelength in the visible range is present around 530 nm. Therefore, in this specification, the color value found from the absorbance at the maximum absorption wavelength around 530 nm is referred to as "color value (530 nm)." Since humans perceive the absorption at a wavelength of 530 nm as bluish red (purple), it is suitable for the evaluation of a purple anthocyanin pigment. More specifically, the "color value (530 nm)" and the content of a purple pigment (anthocyanin pigment) are correlative. A high color value means a high purple color pigment content (anthocyanin pigment content).

Specifically, the measurement of the color value (530 nm) is performed by measuring the absorbance of a test sample at the maximum absorption wavelength around a wavelength of 530 nm with an optical light path of 10 mm using a spectrophotometer (a V-560 (JASCO Corporation) or a similar product). The test sample used herein for the absorbance measurement is a test liquid adjusted so that the absorbance falls within a range of 0.3 to 0.7.

The test liquid used for the measurement of color value (530nm)/g of the colored sweet potato of the present invention is prepared as follows.

- Method for Preparing Test Liquid

About 50 g of the target colored sweet potato (raw) was cut into 5-mm squares, and accurately weighed (weighed amount $\alpha$g). Then, the measured sample was placed in a beaker, and a 0.5 w/w% sulfuric acid aqueous solution was added thereto so that the entire amount was about 250 g (the entire solid-liquid amount $\beta$g). The sample was then subjected to immersion in a dark place at room temperature (25$\pm$5°C) for one day (about 24 hours), thereby extracting a pigment component. Subsequently, the resulting mixture was filtrated with a filter paper (ADVANTEC NO.5C) (solid-liquid separation), and the filtrate was appropriately diluted with McIlvaine's buffer (pH 3.0) ($\gamma$-fold) so as to adjust the absorbance within a range of 0.3 to 0.7. The obtained diluent is used as a test liquid.

- Method for Measuring Color Value (530nm)/g

The absorbance $A_{530}$ of the test liquid thus prepared at the maximum absorption wavelength around a wavelength of 530 nm was measured using an ultraviolet and visible spectrophotometer. Further, so as to correct the influence of the possible residual turbidity after the filtration to the absorbance, absorbance $A_{700}$ (turbidity index) at a wavelength of 700 nm is measured as well.

The color value (530 nm) per gram of wet weight of the colored sweet potato (color value (530nm)/g) may be calculated from a measurement value obtained by the method described above according to the following formula.

$$\text{Color value (530nm)/g} = (A_{530} - A_{700}) \times \gamma \times \beta \div \alpha \div 10$$

$A_{530}$ = Absorbance of test liquid at the maximum absorption wavelength around a wavelength of 530 nm
$A_{700}$ = Absorbance of test liquid at a wavelength of 700 nm
$\Gamma$ = dilution rate adjusted so that the measured absorbance falls within a range of 0.3 to 0.7.
B = Entire amount of colored sweet potato (wet weight) and extraction solvent (entire solid-liquid amount)
$\alpha$ = wet weight of colored sweet potato

10 = value used for the conversion to the absorbance of a 10 w/v% solution.

The magnitude of the "color value (530nm)/g" thus calculated reflects the degree of the amount of the pigment component of the purple color contained in the colored sweet potato (tuberous root), thereby appropriately indicating the characteristics and capability of the colored sweet potato as a purple color pigment material.

The colored sweet potato of the present invention is characterized in that the color value (530nm)/g measured by the above method is generally not less than 15. The color value (530nm)/g is preferably not less than 16, more preferably not less than 18, further preferably not less than 20, particularly preferably not less than 22. The upper limit is not particularly restricted, but is generally not more than 40, particularly not more than 30. In contrast, as shown in Example 2, the color values (530 nm) of *Ayamurasaki, Akemurasaki,* and *Murasakimasari,* which are existing varieties of colored sweet potato, are generally not more than 12, which is significantly lower than the color value (530 nm) of the colored sweet potato of the present invention. From this viewpoint, the colored sweet potato of the present invention is clearly different from existing colored sweet potatoes.

*Ayamurasaki, Akemurasaki,* and *Murasakimasari,* which are existing varieties of colored sweet potato, are commercially available from Sanwa Green, Ueyama Shubyo Corporation, Anet Corporation, and the like (NPL 1).

<u>(B) The absorbance ratio (320nm/530nm) per gram of wet weight of the colored sweet potato is not less than 1.5.</u>

In the present invention, the "absorbance ratio (320nm/530nm)" means a ratio of "absorbance $A_{320}$ at the maximum absorption wavelength around a wavelength of 320 nm" to the "absorbance $A_{530}$ at the maximum absorption wavelength around a wavelength of 530 nm" ("absorbance $A_{320}$ at the maximum absorption wavelength around a wavelength of 320nm/absorbance $A_{530}$ at the maximum absorption wavelength around a wavelength of 530 nm").

Absorbance $A_{530}$ correlates with the content of the purple color pigment (such as anthocyanin pigment); a high absorbance means a high purple color pigment content. On the other hand, absorbance $A_{320}$ correlates with the content of a polyphenol such as chlorogenic acid; a high absorbance means a high polyphenol content. The "absorbance ratio (320nm/530nm)," which corresponds to the ratio of these two absorbances, means a ratio of "absorbance of polyphenol" to "absorbance of purple pigment." The value of not less than 1.5 per gram of wet weight of the colored sweet potato (this may also simply be referred to as "absorbance ratio (320nm/530nm)/g" in the present invention) means a higher polyphenol content relative to the amount of the purple pigment contained in the colored sweet potato of the present invention. Since polyphenols have antioxidant effects, this indicates that the colored sweet potato has higher antioxidant effects relative to the amount of the purple color pigment contained therein.

Specifically, the measurements of "absorbance $A_{530}$ at the maximum absorption wavelength around a wavelength of 530 nm" and "absorbance $A_{320}$ at the maximum absorption wavelength around a wavelength of 320 nm" in the target colored sweet potato are performed, specifically, by measuring absorbance $A_{530}$ and absorbance $A_{320}$ of the test sample at the maximum absorption wavelengths around wavelengths of 530 nm and 320 nm with an optical light path of 10 mm using a spectrophotometer (a V-560 (JASCO Corporation), or a similar product), as in the measurement of the color value (530 nm). The test sample used for the absorbance measurement is a test liquid adjusted so that the absorbance falls within a range of 0.3 to 0.7.

The test liquid used for the measurement of absorbance $A_{530}$ and absorbance $A_{320}$ of the colored sweet potato of the present invention may be prepared by a method similar to that for the test liquid used for the measurement of the color value (530 nm).

Using an ultraviolet and visible spectrophotometer, the absorbance $A_{530}$ at the maximum absorption wavelength around a wavelength of 530 nm and absorbance $A_{320}$ at the maximum absorption wavelength around a wavelength of 320 nm of the prepared test liquid are measured; and absorbance $A_{700}$ at a wavelength of 700 nm is also measured so as to correct the influence of the possible residual turbidity, which may slightly remain after the filtration, to the absorbance.

The absorbance ratio (320nm/530nm) per gram of wet weight of colored sweet potato (absorbance ratio (320nm/530nm)/g) may be calculated from measurement values ($A_{320}$, $A_{530}$, $A_{700}$) obtained by the method described above according to the following formula.

$$\text{Absorbance ratio (320nm/530nm)/g} = (A_{320} - A_{700}) \div (A_{530} - A_{700})$$

$A_{530}$= Absorbance of test liquid at the maximum absorption wavelength around a wavelength of 530 nm
$A_{320}$= Absorbance of test liquid at the maximum absorption wavelength around a wavelength of 320 nm
$A_{700}$ = Absorbance of test liquid at a wavelength of 700 nm

The magnitude of the "absorbance ratio (320nm/530nm)/g" thus calculated reflects the degree of antioxidant effects (antioxidant performance) per unit amount of the pigment component of the purple color contained in the colored sweet potato (tuberous root), thereby appropriately indicating the characteristics and capability of the colored sweet potato as a purple color pigment material, i.e., the capability of stably retaining the pigment based on the antioxidant capability of the colored sweet potato itself.

The colored sweet potato of the present invention is characterized in that the absorbance ratio (320nm/530nm)/g measured by the above method is generally not less than 1.5. The absorbance ratio (320nm/530nm)/g is preferably not less than 1.8, more preferably not less than 2. The upper limit is not particularly restricted, but is generally, for example, not more than 4, particularly not more than 3.

(C) Color value (530nm)/g × absorbance ratio (320nm/530nm)/g = not less than 30.

The value can be calculated by multiplying "color value (530nm)/g" found in (A) above by "absorbance ratio (320nm/530nm)/g" found in (B) above. The value serves as an index of the amount of a stabilizing component (e.g., a polyphenol such as chlorogenic acid) contained in the colored sweet potato.

The colored sweet potato of the present invention is characterized in that the value of "color value (530nm)/g" × "absorbance ratio (320nm/530nm)/g" is not less than 30. The value is preferably not less than 33, more preferably not less than 40, further preferably not less than 42, particularly preferably not less than 45. The upper limit is not particularly restricted, but is generally, for example, not more than 70, particularly not more than 65. In contrast, as shown in Example 2, the values of *Ayamurasaki, Akemurasaki,* and *Murasakimasari,* which are existing varieties of colored sweet potato, are generally less than 30, which is significantly lower than that of the colored sweet potato of the present invention. From this viewpoint, the colored sweet potato of the present invention is clearly different from the existing varieties of colored sweet potatoes.

The colored sweet potato of the present invention is characterized by at least having characteristics (A) to (C) described above, and also includes those having characteristic (D) below (for ease of explanation, this colored sweet potato may hereinafter also be referred to as "colored sweet potato D"), in addition to characteristics (A) to (C); or those having characteristic (E) below (for ease of explanation, this colored sweet potato may hereinafter also be referred to as "colored sweet potato E"), in addition to characteristics (A) to (C). Further, the colored sweet potato of the present invention also includes a sweet potato having all of characteristics (A) to (E). For ease of explanation, this colored sweet potato may hereinafter also be referred to as "colored sweet potato DE."

(D) Insertion of LTR retrotransposon Rtsp-1 (CL1836 and CL1056)

LTR retrotransposon (hereinafter may also be simply referred to as "Rtsp-1") is LTR retrotransposon having a repetitive sequence called an LTR (Long Terminal Report) at both terminals of the sequence. More specifically, as shown in Fig. 1, LTR is present at each terminal, and PBS (primer binding site) and PPT (poly purine truct), which are sequences necessary in the reverse transcription, are present immediately adjacent the LTR at the five-prime end and the LTR at the three-prime end in the inner portion thereof. This characteristic enables, even when the entire-length sequence of retrotransposon is unknown, identification of the LTR sequence in the direction extended from the five-prime end or the three-prime end thereof, as well as the genomic sequence adjacent to the LTR sequence, by identifying the sequence of the above-described reverse transcription regions using the TAIL-PCR method or the suppression PCR method.

The colored sweet potatoes D and DE of the present invention are characterized in that Rtsp-1 is inserted into at least two positions (CL1836 and CL1056) of the genome sequence thereof. In the genome sequence of the colored sweet potatoes D and DE of the present invention, the insertion of Rtsp-1 into CL1836 and CL1056 may be confirmed by performing a nucleic acid amplification reaction using, as the template DNA, genomic DNA prepared from a plant body of the colored sweet potatoes D and DE of the present invention and a primer set having a combination of a primer (adjacent primer) designed based on the base sequence of the genomic region adjacent to Rtsp-1 to be inserted and a primer designed based on the base sequence of Rtsp-1 to be inserted, and confirming the production of the amplified product as an index. More specifically, for the test samples (genomic DNA) prepared from plants such as sweet potato, it is possible to determine that the target test sample is derived from colored sweet potato D or DE of the present invention (i.e. the target plant corresponds to the plant of colored sweet potato D or DE of the present invention) when the amplified product was confirmed after the nucleic acid amplification reaction; and it is also possible to determine that the target test sample is not derived from any of colored sweet potatoes D and DE of the present invention (i.e. the target plant does not correspond to the plant of colored sweet potato D or DE of the present invention) when the amplified product was not confirmed after the nucleic acid amplification reaction.

Further, if the test sample is an edible composition (for example, a colorant as a food additive, a processed food, and the like), when the production of amplified product was confirmed after the nucleic acid amplification reaction using the edible composition, it is possible to determine that the colored sweet potato D or DE of the present invention is used as the raw material of the edible composition.

The plant body used herein for the preparation of genomic DNA may be any portion of a plant of colored sweet potato (leaves, stems, tuberous roots, etc.), and is not particularly limited. The plant is preferably a fresh leaf.

The DNA extraction may be performed by any previously known method for extracting genomic DNA from a plant. For example, the extraction may be performed with reference to the CTAB (cetyltrimethyl ammonium bromide) method (Tobacco DNA/RNA isolation method, Takahiko Hayakawa (1997), "New Edition of Plant PCR Experiment Protocol" pp. 49-56, Isao Shimamoto, supervised by Takuji Sasaki, Shujunsha), Manual of Assessment and Analysis for Genetically Modified Foods (JAS Analytical Test Handbook 2002), and the like.

The primer set used for the nucleic acid amplification reaction is not limited, and any primer set may be used as long as the above object can be achieved. Examples include those listed in the table below.

Table 1

| Primer set 1 for Rtsp-1 insertion/detection in CL1836 |
|---|
| Forward Primer: SEQ ID NO:1<br>ppt Primer: 5-ATCTAATCTTCAAGTGGGAGATTGTCG-3'<br>Reverse Primer: SEQ ID NO:2<br>CL1836 Primer: 5'-GGfCCAATGCAAGfAAGGfATACAACTTAAACCTCTTATGfCfATGAAGT-3' |
| Primer set 2 for Rtsp-1 insertion/detection in CL1056 |
| Forward Primer: SEQ ID NO:1<br>ppt Primer: 5-ATCTAATCTTCAAGTGGGAGATTGTCG-3'<br>Reverse Primer: SEQ ID NO:3<br>CL1056 Primer: 5'-GAAACACTTGATGTGAACTCCACAACATGATGAGAATTACTTGTGGCAAC-3' |

The "ppt primer" is a primer designed based on the base sequence of the PPT region of Rtsp-1 to be inserted. Further, "CL1836 primer" and "CL1053 primer" correspond to the adjacent primers described above. The adjacent primers can be designed based on the base sequences of the genomic regions respectively adjacent to the two Rtsp-1 (CL1836, CL1056) inserted into the colored sweet potato of the present invention. However, the adjacent primers are preferably designed based on the base sequence of a highly specific part among the base sequences in the region.

As shown in primer sets 1 and 2, the forward primer (ppt primer) and the reverse primer (CL1836 primer, CL1056 primer) are designed so that, in the Rtsp-1 insertion site, an amplified product of about 500 to 530bp is generated between itself and the PPT sequence in the inner portion thereof. More specifically, an amplified product having 522bp base sequence may be obtained by performing a nucleic acid amplification reaction using, as a test sample, genomic DNA of colored sweet potato D or DE of the present invention using primer set 1 having ppt primer (SEQ ID NO:1) and CL1836 primer (SEQ ID NO:2). On the other hand, an amplified product having 512bp base sequence may be obtained by performing a nucleic acid amplification reaction using, as a test sample, genomic DNA of colored sweet potato D or DE of the present invention using primer set 2 having ppt primer (SEQ ID NO:1) and CL1056 primer (SEQ ID NO:3).

The nucleic acid amplification reaction may be performed by appropriately selecting a previously known nucleic acid amplification means. Specifically, examples of the method include the PCR method, multiplex-PCR method, ICAN method, UCAN method, LAMP method, primer extension method, and the like. Among these, the PCR method and multiplex-PCR method are preferably used.

Examples of the method for confirming the presence or absence of amplified product include, but are not limited to, electrophoresis. The electrophoresis may be any method that is not to detect the difference in length of the amplified product, but to detect the presence or absence of the amplified product having the approximate desired size (base length), specifically about 500 to 530bp.

Therefore, for example, agarose gel electrophoresis, polyacrylamide electrophoresis, and like simple electrophoresis may be used according to conventional methods. More specifically, an amplified product having about 522bp base length and an amplified product having about 512bp base length may be confirmed by electrophoresis when a nucleic acid amplification reaction is performed using primer set 1 or 2; and using, as a test sample, genomic DNA of the colored sweet potato of the present invention.

The size (base length) of the amplified product may vary as appropriate according to the design of the primer set. For example, the amplified fragment length becomes longer when the reverse primer is designed at a position more distant from ppt primer corresponding to the forward primer; in contrast, the amplified fragment length becomes shorter when the reverse primer is designed at a position closer to ppt primer.

Examples of the amplified fragment length include, but are not limited to, about 40bp to 1000bp, preferably 40bp to 850bp, more preferably 40bp to 700bp. The primer set is preferably designed so that the amplified product has a base length in this range.

The insertion of Rtsp-1 into CL1836 and CL1056 is accepted only for, in addition to the colored sweet potato of the present invention, the mother strain sweet potato (*Kyukei* 04208-2), which is a crossing parent of the colored sweet potato of the present invention, among the existing sweet potato varieties. The mother strain sweet potato (*Kyukei* 04208-2) is a sweet potato variety for use in seed potatoes stored in the Kyushu Okinawa Agricultural Research Center (National Agriculture and Food Research Organization), and is not commercially used as food (including use as food and food additives). In the present invention, "food or edible" means products that are intended to be commercially used as a food or food additive.

Further, the content of the purple color pigment in the inner tissue of the tuberous root of the sweet potato (*Kyukei* 04208-2) is small; the color value (530 nm) per gram of wet weight of the sweet potato (color value (530nm)/g) is less than 15. This reveals that the colored sweet potato (*Kyushu* No.180) of the present invention is a unique colored sweet potato in which Rtsp-1 is inserted into at least one of CL1836 and CL1056 of the genome sequence, among various edible colored sweet potatoes.

Further, among various colored sweet potatoes, including not only edible colored sweet potatoes but also non-food colored sweet potatoes such as *Kyukei* 04208-2 described above, the colored sweet potato (*Kyushu* No.180) of the present invention is a unique colored sweet potato that satisfies characteristics (A) to (C), as well as characteristic (D).

As described above, since the insertion of Rtsp-1 into at least one of those positions (CL1836 and CL1056) is the unique characteristic of the colored sweet potato of the present invention compared with general edible colored sweet potatoes, this characteristic may be effectively used as the indicator (index) in the screening of the colored sweet potato of the present invention among various edible colored sweet potatoes.

Further, this characteristic may also be effectively used as the indicator (index) in the determination of the variety of the colored sweet potato contained as a raw material in a colorant (pigment composition) prepared from edible colored sweet potato or an edible composition containing edible colored sweet potato, such as food.

The primer set for use in the nucleic acid amplification reaction for confirming the Rtsp-1 insertion inherent in the colored sweet potato of the present invention is not limited to the above two types; for example, primer sets 3 to 9 in the following tables may also be used.

Table 2

| Primer set 3 for Rtsp-1 insertion/detection |
|---|
| Forward Primer: SEQ ID NO:1<br>ppt Primer: 5'-ATCTAATCTTCAAGTGGGAGATTGTCG-3'<br>Reverse Primer: SEQ ID NO:4<br>CL103 Primer: 5'-GAAGTTGCCCAAACAATGCAATCAGC-3' |
| Primer set 4 for Rtsp-1 insertion/detection |
| Forward Primer: SEQ ID NO:1<br>ppt Primer: 5'-ATCTAATCTTCAAGTGGGAGATTGTCG-3'<br>Reverse Primer: SEQ ID NO:5<br>Pattern228 Primer: 5'-CACAATGCCTTCATTGTCTTGAACCC-3' |
| Primer set 5 for Rtsp-1 insertion/detection |
| Forward Primer: SEQ ID NO: 1<br>ppt Primer: 5'-ATCTAATCTTCAAGTGGGAGATTGTCG-3'<br>Reverse Primer: SEQ ID NO:6<br>Pattern238 Primer: 5-GTTGGCTGCTCAACCTCAGTAGC-3' |
| Primer set 6 for Rtsp-1 insertion/detection |
| Forward Primer: SEQ ID NO:1<br>ppt Primer: 5'-ATCTAATCTTCAAGTGGGAGATTGTCG-3'<br>Reverse Primer: SEQ ID NO:7<br>Pattern264 Primer: 5'-CCAATGTGCGAAGGCACTACTCC-3' |

Table 3

| Primer set 7 for Rtsp-1 insertion/detection |
|---|
| Forward Primer: SEQ ID NO:1 |

(continued)

| Primer set 7 for Rtsp-1 insertion/detection |
|---|
| ppt Primer: 5'-ATCTAATCTTCAAGTGGGAGATTGTCG-3'<br>Reverse Primer: SEQ ID NO:8<br>Pattern275 Primer: 5'-GGACCAATGCTGGGACAAGGTC-3' |
| Primer set 8 for Rtsp-1 insertion/detection |
| Forward Primer: SEQ ID NO:1<br>ppt Primer: 5'-ATCTAATCTTCAAGTGGGAGATTGTCG-3'<br>Reverse Primer: SEQ ID NO:9<br>Pattern290 Primer: 5-CCTCGAGCTGCTAAAGTACTTATTGG-3' |
| Primer set 9 for Rtsp-1 insertion/detection |
| Forward Primer: SEQ ID NO:1<br>ppt Primer: 5-ATCTAATCTTCAAGTGGGAGATTGTCG-3'<br>Reverse Primer: SEQ ID NO:10<br>P-CL121 Primer: 5'-CATTTCCACCGTCCAGGAGCC-3' |

(E) The total amount of the aroma components per color value (530 nm) is not more than 40%, based on the total amount of the aroma components per color value (530 nm) of the existing variety *Akemurasaki.*

This indicates a small amount of aroma components in colored sweet potatoes E and DE of the present invention. More specifically, this indicates a small amount of aroma components per purple pigment contained in colored sweet potatoes E and DE.
The target aroma components are 23 kinds of aroma components including butanol, isoamyl alcohol, acetoin, acetol, nonanal, acetic acid, 2-ethylhexanol, benzaldehyde, isobutyric acid, $\gamma$-butyrolactone, menthol, $\alpha$-terpineol, guaiacol, phenethyl alcohol, 3,7-dimethyloct-1-en-3,7-diol, 3-hydroxy-2-pyrone, pantolactone, cis-1,8-terpin, 4-vinylguaiacol, 5-(hydroxymethyl)furfural, vanillin, zingerone, and vanillyl alcohol.
The amounts of the aroma components contained in colored sweet potatoes E and DE may be found by GC/MS analysis. The preparation of the sample subjected to the GC/MS analysis (GC/MS measurement sample), and the GC/MS analysis conditions are described below.

- Preparation of GC/MS measurement sample

About 50g of colored sweet potato (tuberous root) is cut into 5 mm squares, and 0.5% sulfuric acid aqueous solution is added thereto so that the entire solid-liquid amount is about 250g. The mixture is subjected to immersion extraction for one day (about 24 hours) at room temperature (25±5°C), followed by filtration using a filter paper (ADVANTEC NO.5C), thereby obtaining a pigment liquid extract. 100 mL of dichloromethane is added to 100 g of the resulting pigment liquid extract, followed by liquid-liquid extraction at room temperature; thereafter, the organic layer is isolated. Then, the organic layer is dehydrated using anhydrous sodium sulfate, and the extract obtained by concentration under reduced pressure using an evaporator is used as a GC/MS measurement sample.

- GC/MS analysis conditions

Gas chromatograph (GC): Agilent 6890N (Agilent Technologies, Inc.)
Mass selective detector (MSD): Agilent 5975 (Agilent Technologies, Inc.)
Column: Agilent J&W DB-WAX (60 m × 0.25 mm) (Agilent Technologies, Inc.)
Oven temperature: 50°C (2 min)→220°C (3°C/min.)
The gas chromatograph, the mass selective detector, and the column are not particularly limited to those above, as long as they ensure equivalent performance.
The total amount of the above 23 kinds of aroma components can be found from the sum of the peak areas of the 23 kinds of aroma components in the GC/MS spectra obtained by the GC/MS analysis.
The amount of the aroma components contained in the tuberous root of the existing variety *Akemurasaki* may also be found in a manner similar to that for colored sweet potatoes E and DE, i.e., by preparing a GC/MS measurement sample according to the method described above and performing the GC/MS analysis under the above conditions.
"The total amount of aroma components per color value (530 nm)" in (E) can be found by finding the color value (530 nm) of the test liquid described in (A), and dividing "the total amount of aroma components" obtained by the method described above by the color value (530 nm) .

The color value (530 nm) of the test liquid may be calculated according to the following formula.

- Method for Measuring Color Value (530 nm) of Test Liquid

Color value (530nm) = $(A_{530nm}-A_{700nm}) \times \gamma \div 10$
$A_{530nm}$ = Absorbance of test liquid at the maximum absorption wavelength around a wavelength of 530 nm
$A_{700nm}$ = Absorbance of test liquid at a wavelength of 700 nm
$\Gamma$ = Dilution rate adjusted so that the measured absorbance falls within a range of 0.3 to 0.7
10 = Value for the conversion to the absorbance of a 10 w/v% solution.

Colored sweet potatoes E and DE of the present invention are characterized in that (E) the value of "the total amount of the aroma components per color value (530 nm)" is not more than 40%, preferably not more than 38%, more preferably not more than 36%, particularly preferably not more than 35%, based on the value of the existing variety *Akemurasaki.* The lower limit is 20%, preferably 10%.

As is clear from the above, in (E), it is not always necessary to find the total amount of the absolute amounts of aroma components to determine "the total amount of aroma components," insofar as the relative ratio with respect to the existing variety *Akemurasaki* can be found. Thus, the sum of the peak areas obtained by the GC/MS analysis may be used as a substitute value for the "total amount of aroma components."

As described above, the colored sweet potato of the present invention is characterized by having characteristics (A) to (D), characteristics (A) to (C) and (E), or characteristics (A) to (E). The colored sweet potato of the present invention is preferably a colored sweet potato having characteristics (A) to (D), more preferably a colored sweet potato having characteristics (A) to (E).

As described in the Examples below, these colored sweet potatoes may be produced by, although not limited to this method, performing hybridization of the mother strain (*Kyukei* 04208-2) and the father strain (*Kyukei* 04222-50); and screening the resulting hybrid individuals based on at least one of characteristics (D) and (E), in addition to at least characteristics (A) to (C).

Each of the mother strain (*Kyukei* 04208-2) and the father strain (*Kyukei* 04222-50) is a sweet potato stored in the Kyushu Okinawa Agricultural Research Center (National Agriculture and Food Research Organization). As with the colored sweet potato of the present invention, the mother strain (*Kyukei* 04208-2) has characteristic (D).

However, as mentioned above, the mother strain sweet potato (*Kyukei* 04208-2) is different from the colored sweet potato of the present invention in that the mother strain is a sweet potato variety for use in seed potato, which is not commercially used as food (including use as food and food additives); and in that it does not satisfy characteristic (A) of the present invention, as its color value (530nm)/g is less than 15. Among the colored sweet potatoes of the present invention, the colored sweet potato having characteristics (A) to (E) is named *"Kyushu* No.180," and is stored in the Kyushu Okinawa Agricultural Research Center (National Agriculture and Food Research Organization).

Insofar as the colored sweet potato of the present invention has characteristics (A) to (D), characteristics (A) to (C) and (E), or characteristics (A) to (E), the colored sweet potato of the present invention is not particularly limited to the sweet potato stored in the Kyushu Okinawa Agricultural Research Center. As shown in Example 9, the colored sweet potato obtained by vegetative propagation (asexual propagation) under normal cultivation conditions using *Kyushu* No.180 as a seed potato having the genetic characteristic of (D) also has characteristics (A) to (C). Therefore, successive-generation products of *Kyushu* No.180 obtained by vegetative propagation are also included in the range of the colored sweet potato of the present invention.

The colored sweet potato of the present invention has a high content of purple pigment (anthocyanin pigment), as clearly shown in characteristic (A); and ensures high antioxidant performance per unit of the purple pigment (anthocyanin pigment), as clearly shown in characteristic (B), thereby ensuring stable content of the pigment. Therefore, the colored sweet potato of the present invention may be useful as a sweet potato for use in a raw material of a purple pigment (anthocyanin pigment).

## (II) Pigment Composition Derived From Colored Sweet Potato and Method for Preparing the Pigment Composition

The present invention further provides a natural pigment composition prepared by using a colored sweet potato as a raw material. The pigment composition is a purple color pigment composition that has a high content of a purple color pigment (anthocyanin pigment) having the maximum absorption wavelength around a wavelength of 530 nm.

More specifically, the pigment composition of the present invention is characterized by containing, as an anthocyanin pigment, at least pigment YGM-0e, pigment YGM-4b, pigment YGM-5a, pigment YGM-6, and pigment YGM-2. The composition of the present invention is also characterized in that the content ratio of each pigment satisfies at least one of (1) to (4) below, as the peak area ratio detected by HPLC under the following conditions:

(1) a/b(YGM-0e/YGM-4b): 0.3 to 2
(2) c/b(YGM-5a/YGM-4b): 2.3 to 10
(3) c/d(YGM-5a/YGM-6): 1.2 to 5
(4) e/a(YGM-2/YGM-0e): 0.1 to 4.5

a: peak area of pigment YGM-0e
b: peak area of pigment YGM-4b
c: peak area of pigment YGM-5a
d: peak area of pigment YGM-6
e: peak area of pigment YGM-2

- Conditions of HPLC Analysis

ODS reverse-phase column (linking group: triacontyl group): pore size (14 nm), specific surface area (300 $m^2/g$), pore volume (1.05 mg/mL), diameter and length (ϕ4.6 × 250nm)
Column temperature: 40°C
Mobile phase: (a) 1v/v% formic acid aqueous solution, (b) acetonitrile

Gradient conditions:

0→15 minutes, (a) 95%→82%, (b) 5%→18%
15→45 minutes, (a) 82%→30%, (b) 18%→70%
45→55 minutes, (a) 30%→20%, (b) 70%→80%
55→60 minutes, (a) 20%→0%, (b) 80%→100%
Flow Rate: 1.0 mL/min
Sample injection amount: 20 μL
Detection: Photodiode array detector (530 nm)

As one example, when JASCO LC-2000Plus series (JASCO Corporation) is used as an HPLC apparatus, and Develosil (registered trademark) C30-UG-5 (ϕ4.6 × 250nm) (Nomura Chemical Co., Ltd., Japan) is used as an ODS column satisfying the above conditions, the peak of pigment YGM-0e is detected at a retention time of about 17.52 minutes, the peak of pigment YGM-4b is detected at a retention time of about 22.07 minutes, the peak of pigment YGM-5a is detected at a retention time of about 22.23 minutes, the peak of pigment YGM-6 is detected at a retention time of about 22.84 minutes, and the peak of pigment YGM-2 is detected at a retention time of about 20.61 minutes.

However, identification of pigment YGM-0e, pigment YGM-4b, pigment YGM-5a, pigment YGM-6, and pigment YGM-2 in HPLC may be performed by a comparison with the profiles of the reference standards of these pigments. Therefore, the HPLC conditions, in particular, the HPLC apparatus, the column, and the detector are not strictly limited to those above, as long as they ensure equivalent performance.

As the area ratio, (1) a/b (YGM-0e/YGM-4b) is 0.3 to 2 as mentioned above, and is preferably 0.3 to 1.5, more preferably 0.3 to 1.2. Further, (2) c/b (YGM-5a/YGM-4b) is 2.3 to 10 as mentioned above, and is preferably 2.4 to 8, more preferably 2.5 to 7.

Further, (3) c/d (YGM-5a/YGM-6) is 1.2 to 5 as mentioned above, and is preferably 1.2 to 3, more preferably 1.2 to 2.5. Further, (4) e/a (YGM-2/YGM-0e) is 0.1 to 4.5 as mentioned above, and is preferably 0.5 to 4, more preferably 0.8 to 3.5.

It is sufficient that the pigment composition of the present invention satisfies at least one of (1) to (4) above; however, the pigment composition of the present invention satisfies preferably 2 or more, more preferably 3 or more, and particularly preferably all, of (1) to (4). The pigment composition of the present invention satisfies at least one of (2) and (4).

When the pigment composition of the present invention satisfies at least two of (1) to (4), the combination may be, for example, a combination of (2) with one of (1), (3), and (4); preferably a combination of (2) and (1), or a combination of (2) and (3). The combination may also be a combination of (4) with one of (1), (2), and (3); preferably a combination of (4) and (1), or a combination of (4) and (3).

The pigment composition of the present invention may contain pigment YGM-0e, pigment YGM-4b, pigment YGM-5a, pigment YGM-6, and pigment YGM-2, as well as at least one anthocyanin pigment among YGM-1a, pigment YGM-1b, pigment YGM-3, and YGM-5b. Examples of preferable pigment compositions include a pigment composition containing pigment YGM-0e, pigment YGM-4b, pigment YGM-5a, pigment YGM-6, and pigment YGM-2; as well as one of (preferably both of) pigment YGM-1a and YGM-1b, and one of (preferably both of) YGM-3 and YGM-5b.

The pigment composition of the present invention includes a pigment composition having a low content of aroma component. This pigment composition may suitably be used as a pigment preparation (dye) itself, or a pigment composition for preparing a pigment preparation (dye). In particular, the pigment composition of the present invention is prepared using an edible colored sweet potato as a raw material, and is therefore useful as a pigment preparation (dye) as a food additive, or a pigment composition for preparing the pigment preparation.

In addition to the above characteristics, the pigment composition is also characterized in that the total amount (concentration) of the aroma components when the color value at the maximum absorption wavelength around a wavelength of 530 nm (may also be referred to as "$E^{10\%}_{lcm}$(530nm)" in the present invention) is 80 ($E^{10\%}_{lcm}$(530nm)=80) is not more than 120 ppm.

"The color value $E^{10\%}_{lcm}$(530nm) at the maximum absorption wavelength around a wavelength of 530 nm" refers to a value obtained by measuring the absorbance of the target pigment composition in the visible range at the maximum absorption wavelength (530 nm) (measurement cell width: 1 cm), and converting the measured absorbance to an absorbance of a solution containing the pigment composition at a concentration of 10w/v%.

The aroma components used herein are 34 kinds of aroma components: 1. ethyl pyruvate, 2. ethyl lactate, 3. trans-linalool oxide (furanoid), 4. ethyl acetoacetate, 5. cis-linalool oxide (furanoid), 6. 2-ethylhexanol, 7. Benzaldehyde, 8. diethyl malonate, 9. ethyl levulinate, 10. gamma-butylolactone, 11. phenylacetaldehyde, 12. beta-angelica lactone, 13. Phenylacetaldehyde DEA, 14.5-ethoxydihydro-2(3H)-furanone, 15. 2(5H)-furanone, 16. ethyl nicotinate, 17.cyclotene, 18. guaiacol, 19.2,3-dihydro-5-hydroxy-6-methyl-(4H)-pyran-4-one, 20. benzyl alcohol, 21. phenethyl alcohol, 22.3,7-dimethyl-1-octadiene-3,7-diol, 23. maltol, 24. 3-hydroxy-2-pyrone, 25. 1-(2-furanyl)-2-hydroxyethanone, 26. cis-1,8-terpin, 27. 4-vinylguaiacol, 28. 3,5-dihydroxy-6-methyl-2,3-dihydro-4(4H)-pyranone, 29. Sulfurol, 30. ethyl vanillyl ether, 31. 5-(hydroxymethyl)furfural, 32. vanillin, 33. ethyl vanillate and 34. vanillyl alcohol. The number in the beginning of the English name of the compound designates a peak number shown in the chromatograms of Fig. 12.

The amounts of these aroma components contained in the pigment composition may be found by GC/MS analysis. The preparation of the sample subjected to the GC/MS analysis (GC/MS measurement sample), and GC/MS analysis conditions are described below.

- Preparation of GC/MS measurement sample

10 μg of 3-heptanol is added as an internal standard substance to a solution obtained by diluting 10 g of the pigment composition with 200 mL of ion-exchanged water. 200 mL of dichloromethane is added to the solution, followed by liquid-liquid extraction at room temperature; thereafter, the organic layer is isolated. The organic layer is dehydrated using anhydrous sodium sulfate, and the extract obtained by concentration under reduced pressure using an evaporator was used as a GC/MS measurement sample.

- GC/MS analysis conditions

Gas chromatograph (GC): Agilent 6890N (Agilent Technologies, Inc.)
Mass selective detector (MSD): Agilent 5975 (Agilent Technologies, Inc.)
Column: Agilent J&W DB-WAX (60 m × 0.25 mm) (Agilent Technologies, Inc.)
Oven temperature: 50°C (2 min)→220°C (3°C/min.)

Example 7 shows a specific example. However, the gas chromatograph, the mass selective detector, and the column are not particularly limited to those above, as long as they ensure equivalent performance.

The total amount of the above 34 kinds of aroma components can be found from the sum of the peak areas of the 34 kinds of aroma components in the GC/MS spectra obtained by the GC/MS analysis. In this manner, the total amount of the aroma components per gram of the pigment composition (concentration: μg/g) is calculated.

The color value "$E^{10\%}_{lcm}$(530nm)" at the maximum absorption wavelength around a wavelength of 530 nm of the pigment composition of the present invention may be found according to the following method.

- Method for Calculating $E^{10\%}_{lcm}$ (530nm)

The pigment composition is appropriately diluted (γ-fold) with McIlvaine's buffer (pH 3.0), and absorbance $A_{530}$ at the maximum absorption wavelength around 530 nm is measured using a V-560 ultraviolet and visible spectrophotometer (JASCO Corporation) (measurement cell width: 1 cm). $E^{10\%}_{lcm}$(530nm) is calculated from the obtained absorbance $A_{530}$ according to the following calculating formula.

$$E^{10\%}_{1cm}(530nm) = A_{530} \times \gamma \div 10$$

10= value for the conversion to the absorbance of a 10 w/v% solution.

The total amount (concentration) of the aroma components when the color value at the maximum absorption wavelength around a wavelength of 530 nm is 80 ($E^{10\%}_{1cm}$(530nm)=80) may be calculated according to the following method.

(The total amount of the aroma components (concentration) in the pigment composition)/Color value at the maximum absorption wavelength around a wavelength of 530 nm of the pigment composition) × 80.

In the pigment composition of the present invention, the total amount (concentration) of the aroma components contained therein when the color value at the maximum absorption wavelength around a wavelength of 530 nm is 80 ($E^{10\%}_{1cm}$(530nm)=80) is not more than 120 ppm, more preferably not more than 100 ppm, further preferably not more than 80 ppm, further more preferably not more than 70 ppm.

The pigment composition of the present invention may be produced and obtained by an extraction treatment of the colored sweet potato of the present invention described above (an extract composition of colored sweet potato; hereinafter may also be referred to as "a pigment liquid extract"). The pigment composition of the present invention may also be produced and obtained by further subjecting the pigment liquid extract obtained by an extraction treatment of the colored sweet potato of the present invention to one of an adsorption treatment, ion-exchange treatment, acid treatment, heating, extraction, and membrane separation; or a combination of two or more of any of these treatments (purified matter). The purified matter may be suitably used, in particular, as a raw material of a pigment preparation.

The pigment liquid extract of colored sweet potato may be prepared by subjecting a colored sweet potato (tuberous root) as is (raw), or a pulverized matter (coarse powder, minced, etc.) thereof, to solvent extraction operation. It is also possible to dry a colored sweet potato; and then, as necessary, pulverize the dried colored sweet potato, and subject the pulverized colored sweet potato in the form of a powder or the like to a solvent extraction operation.

The solvent used for the extraction is not particularly limited, and water, alcohol, or a mixture thereof may preferably be used. Examples of alcohol include lower alcohols having 1 to 4 carbon atoms, such as methanol, ethanol, propanol, isopropyl alcohol, or butanol. Water or hydrous alcohol is preferable. The hydrous alcohol is preferably a hydrous alcohol having an alcohol content of not more than 60 volume%.

Further, as the solvent used for the extraction, an acid solution, more specifically, an acid solution adjusted to have a pH of 1 to 4, preferably 1 to 3, may be used. The acid solution may be prepared by incorporating hydrochloric acid, sulfuric acid, nitric acid, phosphoric acid and like inorganic acids; or citric acid, acetic acid, malic acid, lactic acid and like organic acids into the extraction solvent. The amount of the inorganic or organic acid to be incorporated into the extraction solvent is not particularly limited insofar as the above pH range is ensured; however, the amount is preferably appropriately adjusted within a range of 0.01 to 10 wt%.

The extraction method may be selected from any methods commonly used. Examples of the method include, but are not limited to; a method of immersing a colored sweet potato (tuberous root) (directly or in the form of a coarse powder, minced matter, or a dried matter thereof) in a solvent by cold extraction, warm extraction, or the like; a method of performing extraction while heating and stirring and then filtration to obtain a liquid extract, a percolation method; and the like. A preferable extraction is extraction under an acidic condition. A method of performing extraction by immersing a minced matter (raw or dried matter, preferably raw) of colored sweet potato in an acid extraction solvent having a pH of 1 to 4 at room temperature is more preferable.

The obtained liquid extract may further be subjected to one of an adsorption treatment, ion-exchange treatment, acid treatment, heating, extraction, and membrane separation, after solids are removed as necessary from the obtained liquid extract by filtration, coprecipitation, or centrifugation.

The adsorption treatment may be performed according to standard methods. Examples include adsorption treatments using activated carbon, silica gel, porous ceramic, or the like; and adsorption treatments using styrene-based Duolite S-861 (registered trademark, Duolite, U.S.A., Diamond Shamrock Corp; the same hereinafter), Duolite S-862, Duolite S-863, or Duolite S-866; aromatic-based Sepabeads SP70 (registered trademark, Mitsubishi Chemical Corporation; the same hereinafter), Sepabeads SP207, Sepabeads SP700, Sepabeads SP825; Diaion HP10 (registered trademark, Mitsubishi Chemical Corporation; the same hereinafter), Diaion HP20, Diaion HP21, Diaion HP40, and Diaion HP50; or Amberlite XAD-4 (registered trademark, Organo Corporation; the same hereinafter), Amberlite XAD-7, Amberlite XAD-2000, and like synthetic adsorption resins.

The pigment component contained in the pigment extract may be collected and obtained by washing a resin carrier in which the pigment component is adsorbed after the adsorption treatment with an appropriate solvent, such as hydrous alcohol. Preferable examples of hydrous alcohol include water containing ethanol in an amount of generally about 1 to 70 volume%, preferably about 20 to 60 volume%, more preferably about 30 to 55 volume%.

The adsorption treatment liquid of the pigment liquid extract of colored sweet potato thus obtained may further be subjected to ion-exchange treatment; and may further be subjected to, as necessary, acid treatment, extraction, membrane separation, or other various treatments.

The ion-exchange treatment is not particularly limited, and may be performed according to standard methods using conventional ion-exchange resins (cation exchange resin or anion exchange resin). Specific examples of cation exchange resins include, but are not limited to, Diaion SK1B (registered trademark, Mitsubishi Chemical Corporation; the same hereinafter), Diaion SK104, Diaion SK110, Diaion PK208, Diaion PK212, Diaion PK216, Diaion WK10, and Diaion WK11; as well as Amberlite IRC76 (registered trademark, Organo Corporation, the same hereinafter), Amberlite FPC3500, and the like. Specific examples of anion exchange resins include, but are not particularly limited to, Diaion SA10A (registered trademark, Mitsubishi Chemical Corporation; the same hereinafter), Diaion SA12A, Diaion SA20A, Diaion PA412, Diaion WA10, Diaion WA20, and the like.

Further, the acid treatment used in the present invention may be performed by exposing the pigment liquid extract of colored sweet potato or the liquid having been subjected to various treatments (solid-liquid separation such as filtration, adsorption treatment, ion exchange treatment, or the like) to an acidic condition of pH 1 to 4, preferably pH 1 to 3. The acid treatment may easily be performed, specifically, by adding an acid to the treatment liquid. The acid is not particularly limited as long as it is generally used as a food additive, and may arbitrarily be selected and used from such additives.

Examples of acids include citric acid, acetic acid, malic acid, lactic acid and like organic acids; and sulfuric acid, hydrochloric acid, phosphoric acid, nitric acid, and like inorganic acids. An acid treatment using an inorganic acid that is generally used as a food additive is preferable. The temperature in the acid treatment is not particularly limited, and may generally be selected and used appropriately from the range of 5 to 100°C.

Examples of the temperature include a range of 20 to 100°C, and a range of 40 to 100°C. The acid treatment time is also not particularly limited, and may generally be selected and used appropriately from the range of 1 to 300 minutes. Generally, the treatment time may be reduced if the acid treatment is performed under a high temperature. For example, when the acid treatment is performed at 40 to 100°C, the treatment time is selected from a range of 5 to 60 minutes. At this time, the treatment may be performed by stirring, or not stirring, the treatment liquid.

The heat treatment used in the present invention may be performed by exposing the pigment liquid extract to a temperature condition of not more than 100°C, specifically a temperature condition in a range of 80 to 95°C. Thereafter, insoluble matter may be removed as necessary.

Further, examples of extraction in the present invention include, but are not particularly limited to, a method in which carbon dioxide, ethylene, propane, or the like in the form of a liquid is brought into contact with a pigment liquid extract of colored sweet potato or a treatment liquid subjected to the above various treatments (adsorption treatment, ion exchange treatment, acid treatment, membrane separation, or the like) in a closed apparatus at a temperature and pressure at or above the critical point.

Further, the membrane separation in the present invention broadly means a filtration method using a membrane. Examples include filtration using a membrane filter (MF) membrane, ultrafiltration (UF) membrane, reverse osmosis membrane (NF), electrodialysis membrane, and like functional polymer membranes.

Further, in addition to the ultrafiltration method and reverse osmosis membrane method using these membranes, dialysis using a concentration gradient with an ion-selective membrane; electrodialysis in which a voltage is applied using an ion exchange membrane as a diaphragm; and the like have been known as membrane separation. Membrane separation using a reverse osmosis membrane method is industrially preferable.

The membrane material used for the membrane separation may be a natural, synthetic, or semisynthetic membrane material. Examples include cellulose, cellulose diacetate or triacetate, polyamide, polysulfone, polystyrene, polyimide, polyacrylonitrile, and the like.

These various treatments may be performed singly, or in any combination of two or more. Further, the same treatment may be repeated under the same or different conditions. A preferred treatment method is, but not particularly limited to, a method of subjecting a pigment liquid extract of colored sweet potato to an adsorption treatment and an ion exchange treatment (cation exchange treatment).

In the colored sweet potato of the present invention, since the content of aroma components contained therein is greatly reduced compared with previously known varieties, the pigment composition derived from the colored sweet potato of the present invention contains a small amount of aroma components derived from colored sweet potato that may cause abnormal odor or unpleasant odor. Therefore, it is possible to provide a natural pigment derived from colored sweet potato that is odorless, or having a slight odor that hardly influences the flavor when the pigment is incorporated in an edible composition such as food or a food additive (e.g., colorant).

The pigment composition derived from the colored sweet potato of the present invention is characterized in that it is relatively stable with respect to light and heat (light resistance, heat resistance); the stability is the same or superior compared with the pigment composition derived from *Ayamurasaki,* which is an existing variety regarded as having relatively excellent light resistance and heat resistance.

The pigment composition of the present invention may be prepared as a pigment preparation in the form of a solution dissolved or dispersed (emulsified) in, preferably, water, ethanol, propylene glycol, or like alcohol; or other solvents; or in a dry state (powder, granules, tablets, pills, and the like). Therefore, the present invention provides a pigment preparation containing the pigment composition derived from colored sweet potato described above.

The pigment preparation may consist only of the components (including pigment components) derived from the colored sweet potato of the present invention. The pigment preparation may also contain carriers or various additives acceptable in terms of food hygiene, in addition to the components (including pigment components) derived from the colored sweet potato of the present invention. Specific examples of the carriers and additives include dextrin, lactose, powdered starch syrup, as well as food additives including preservatives (sodium acetate, protamine, or the like), stabilizers (sodium phosphate, sodium metaphosphate, or the like), and antioxidants (rutin, ascorbic acid, or the like), which are generally used for pigments or pigment preparations.

When the pigment preparation of the present invention contains various carriers and additives, the proportion of the pigment composition made of the pigment component derived from colored sweet potato contained in the pigment preparation is, for example, but not particularly limited to, generally 1 to 90 wt%, preferably 10 to 60 wt%. The pigment preparation of the present invention is useful as a purple colorant, in particular, as a natural purple colorant for foods, pharmaceuticals, quasi-drugs, cosmetics, feeds, and the like. The coloring of food and beverage not only includes coloring by artificial addition of a pigment preparation to a food or beverage, but also broadly includes coloring by using the colored sweet potato of the present invention for a material of food or beverage.

(III) Method for Determining Variety of Sweet Potato, and Reagent Used for the Method

As described above, the colored sweet potato of the present invention is a unique edible colored sweet potato in which Rtsp-1 is inserted in one of CL1836 and CL1056 of the genomic DNA sequence. "Edible" herein means use as a food and a food additive. Examples of the use as a food additive include pigment preparations (colorants, dyes).

This indicates that the colored sweet potato of the present invention may be screened from a plurality of edible colored sweet potatoes using the insertion of Rtsp-1 into the site as an indicator (index). Further, this indicator (index) may also be effectively used as the indicator (index) in the determination of the variety of an edible colored sweet potato contained as a raw material in an edible composition (food additive such as a colorant (pigment composition), processed food, and the like) prepared from edible colored sweet potato.

Therefore, the present invention provides a method for determining a variety of an edible colored sweet potato, or an edible colored sweet potato used as a raw material of an edible composition containing, as a raw material, an edible colored sweet potato. The method may be performed by a method comprising the following steps (1) and (2), or a method comprising the following steps (1') and (2').

(1) a step of performing a nucleic acid amplification reaction using, as a template, DNA prepared from an edible colored sweet potato or an edible composition containing an edible colored sweet potato, and at least one of primer set 1 containing a forward primer comprising the base sequence of SEQ ID NO:1 and a reverse primer comprising the base sequence of SEQ ID NO:2, and primer set 2 containing a forward primer comprising the base sequence of SEQ ID NO:1 and a reverse primer comprising the base sequence of SEQ ID NO:3; and
(2) a step of confirming the presence or absence of production of amplified product having a fragment length of 500 to 530bp by the nucleic acid amplification reaction.

(1') a step of performing a nucleic acid amplification reaction using, as a template, DNA prepared from an edible colored sweet potato or an edible composition containing an edible colored sweet potato, and at least one primer set selected from the group consisting of primer sets 3 to 9 containing a forward primer comprising the base sequence of SEQ ID NO:1 and a reverse primer comprising the base sequence of any one of SEQ ID NOs:4 to 10; and
(2') a step of confirming the presence or absence of production of amplified product having a fragment length of 550 to 650bp by the nucleic acid amplification reaction.

In step (1) or step (1'), the part of the sweet potato used for the preparation of genomic DNA may be any portion of a plant thereof (leaves, rhizome, tuberous roots, etc.), and is not particularly limited. The part is preferably a fresh leaf. The DNA extraction may be performed by any previously known method for extracting genomic DNA from a plant, if the colored sweet potato itself is the target.

For example, the extraction may be performed with reference to the CTAB (cetyltrimethyl ammonium bromide) method (Tobacco DNA/RNA isolation method, Takahiko Hayakawa (1997), "New Edition of Plant PCR Experiment Protocol" pp. 49-56, Isao Shimamoto, supervised by Takuji Sasaki, Shujunsha), and the like. Further, when an edible composition (e.g., a processed food) prepared by using colored sweet potato as a raw material is the target, the extraction may be performed with reference to, for example, the Manual of Assessment and Analysis for Genetically Modified Foods (JAS Analytical Test Handbook 2002), and the like.

The forward primer (ppt primer) and the reverse primer (CL1836 primer, CL1056 primer) constituting primer sets 1 and 2 used for the nucleic acid amplification reaction described above are, as described in Item (I)(D) above, designed so that in the Rtsp-1 insertion site, an amplified product of about 500 to 530bp is generated between itself and the PPT sequence in the inner portion thereof. More specifically, an amplified product having 522bp base sequence may be obtained by performing a nucleic acid amplification reaction using, as a template, genomic DNA extracted and prepared from the target test sample and primer set 1 having ppt primer (SEQ ID NO:1) and CL1836 primer (SEQ ID NO:2) described above.

In contrast, an amplified product having 512bp base sequence may be obtained by performing a nucleic acid amplification reaction using, as a template, genomic DNA extracted and prepared from the target test sample and primer set 2 having ppt primer (SEQ ID NO:1) and CL1056 primer (SEQ ID NO:3) described above. The nucleic acid amplification reaction may be performed by appropriately selecting a previously known nucleic acid amplification means, as mentioned above.

The forward primer (ppt primer) and reverse primers 3 to 9 (CL103 primer, Pattern 228 primer, Pattern 238 primer, Pattern 264 primer, Pattern 275 primer, Pattern 290 primer, and CL121 primer) constituting primer sets 3 to 9 used for the nucleic acid amplification reaction described above are designed so that, in the Rtsp-1 insertion site, an amplified product of about 500 to 530bp is generated between itself and the PPT sequence in the inner portion thereof. More specifically, an amplified product having the base sequence of the following base length may be obtained by performing a nucleic acid amplification reaction using, as a template, genomic DNA extracted and prepared from the target test sample and these primer sets 3 to 9.

Table 4

| Primer Set | Forward Primer | Reverse Primer | Base Length of Amplified Product (bp) |
|---|---|---|---|
| 3 | ppt Primer (SEQ ID NO: 1) | CL103 Primer (SEQ ID NO:4) | 600 |
| 4 | ppt Primer (SEQ ID NO: 1) | Pattern228 Primer (SEQ ID NO: 5) | 581 |
| 5 | ppt Primer (SEQ ID NO: 1) | Pattern238 Primer (SEQ ID NO: 6) | 641 |
| 6 | ppt Primer (SEQ ID NO: 1) | Pattern264 Primer (SEQ ID NO: 7) | 622 |
| 7 | ppt Primer (SEQ ID NO: 1) | Pattern275 Primer (SEQ ID NO: 8) | 610 |
| 8 | ppt Primer (SEQ ID NO: 1) | Pattern290 Primer (SEQ ID NO: 9) | 620 |
| 9 | ppt Primer (SEQ ID NO: 1) | CL121 Primer (SEQ ID NO:10) | 571 |

All of these primer sets 1 to 9 are useful as a component of a reagent for determining a variety of a colored sweet potato for use in the variety determination method of the present invention. The present invention provides these primer sets. To confirm the Rtsp-1 insertion into both CL1836 and CL1056 of the genomic DNA sequence of the target colored sweet potato, both primer sets 1 and 2 may preferably be used as a primer for a nucleic acid amplification reaction.

In step (2) or step (2'), examples of the method for confirming the presence or absence of amplified product include, but are not limited to, electrophoresis. The electrophoresis may be any method that is not to detect the difference in length of the amplified product, but to detect the presence or absence of the amplified product having the approximate desired size (base length), specifically about 500 to 650bp.

Therefore, for example, agarose gel electrophoresis, polyacrylamide electrophoresis, and like simple electrophoresis may be used according to conventional methods. More specifically, when a nucleic acid amplification reaction is performed using one of primer sets 1 to 9, and when the test sample is the colored sweet potato of the present invention or when the test sample contains the colored sweet potato of the present invention, it is possible to confirm production of an amplified product having a base length corresponding to the primer set used by electrophoresis.

The variety determination method of the present invention may comprise a step of determining that a colored sweet potato used as the test sample or a colored sweet potato contained in an edible composition used as the test sample is the colored sweet potato of the present invention when production of an amplified product is confirmed in step (2) or step (2').

More specifically, when an amplified product having a base length corresponding to the primer set used is confirmed after the nucleic acid amplification reaction described above is performed using a test sample (genomic DNA) prepared from a plant of an edible colored sweet potato, it is possible to determine that the target test sample is the colored sweet potato of the present invention (i.e. the target plant corresponds to the plant of the colored sweet potato of the present invention); and it is also possible to determine that the target test sample is not derived from the colored sweet potato of the present invention (i.e. the target plant does not correspond to the plant of the colored sweet potato of the present invention), when the amplified product is not confirmed after the nucleic acid amplification reaction. Further, if the test sample is an edible composition (for example, a colorants as a food additive, a processed food, or the like), it is possible to determine that the colored sweet potato of the present invention is used as the raw material of the edible composition when the production of an amplified product is confirmed after the nucleic acid amplification reaction using the edible composition.

As described above, the following primer sets provided by the present invention are useful as a component of a reagent for determining a variety of an edible colored sweet potato for use in the variety determination method of the present invention. The present invention provides a kit for determining a variety of colored sweet potato comprising at least one, preferably two or more, of these primer sets. Preferable examples of two or more primer sets include the combination of primer set 1 and primer set 2.

(a) primer set 1 containing a forward primer comprising the base sequence of SEQ ID NO:1 and a reverse primer comprising the base sequence of SEQ ID NO:2,
(b) primer set 2 containing a forward primer comprising the base sequence of SEQ ID NO:1 and a reverse primer comprising the base sequence of SEQ ID NO:3,
(c) primer set 3 containing a forward primer comprising the base sequence of SEQ ID NO:1 and a reverse primer comprising the base sequence of SEQ ID NO:4,
(d) primer set 4 containing a forward primer comprising the base sequence of SEQ ID NO:1 and a reverse primer comprising the base sequence of SEQ ID NO:5,
(e) primer set 5 containing a forward primer comprising the base sequence of SEQ ID NO:1 and a reverse primer comprising the base sequence of SEQ ID NO:6,
(f) primer set 6 containing a forward primer comprising the base sequence of SEQ ID NO:1 and a reverse primer comprising the base sequence of SEQ ID NO:7,
(g) primer set 7 containing a forward primer comprising the base sequence of SEQ ID NO:1 and a reverse primer comprising the base sequence of SEQ ID NO:8,
(h) primer set 8 containing a forward primer comprising the base sequence of SEQ ID NO:1 and a reverse primer comprising the base sequence of SEQ ID NO:9,
(i) primer set 9 containing a forward primer comprising the base sequence of SEQ ID NO:1 and a reverse primer comprising the base sequence of SEQ ID NO:10.

The kit may comprise, in addition to the primers, reagent or tools for use in the extraction of DNA from the target test sample (plant of colored sweet potato, edible composition, or the like) or purification thereof; reagent or tools for use in a nucleic acid amplification reaction; reagent or tools for use in electrophoresis; and the like.

Examples

The features and the effects of the present invention are explained below based on Examples. However, those shown below are examples merely to demonstrate the features and the effects of the present invention, and the present invention is not limited to the following Examples.

Example 1: Production of a Variety of Colored Sweet Potato Having High Anthocyanin Content

Hybridization of mother sweet potato *Kyukei* 04208-2 and father sweet potato *Kyukei* 04222-50 was performed. Both the mother variety and the father variety are stored in the Kyushu Okinawa Agricultural Research Center (National Agriculture and Food Research Organization).

More specifically, the hybridization was performed according to the following procedures. The seeds of a bush morning glory for rootstock were subjected to a sulfuric acid treatment to overcome the hardseededness; and were made to absorb water, followed by seeding. They were grown for a month or two after the seeding until about 15 to 20 true leaves were grown. Thereafter, the stem of the bush morning glory was cut off while leaving about 10 true leaves, and sweet potato cut into a wedge shape was grafted by cleft grafting. After fixation with clips, the plant was covered with a plastic bag.

After blooming, the anther of the father variety was pinched with tweezers, and was pressed lightly to the stigma of the pistil of a pre-emasculated mother variety so as to perform hybridization. Thereafter, the obtained seeds were planted in a farm field, and the superior strains were screened based on the yield, disease resistance, color value, absorbance ratio, and like data.

After the hybridization, a part of the tuberous root (the potato portion) of the obtained individual was collected, and the absorbance at a maximum absorption wavelength around a wavelength of 530 nm (color value (530 nm)) and the absorbance at a maximum absorption wavelength around a wavelength of 320 nm (color value (320 nm)) were analyzed/determined using a spectrophotometer. The results determined that, unexpectedly, both the color value (530 nm) per gram of wet weight of colored sweet potato (color value (530 nm)/g) and color value (320 nm) per gram of wet weight of colored sweet potato (color value (320 nm)/g) were increased.

The color value (530 nm)/g reflects an anthocyanin pigment content.

Further, the absorbance at a wavelength of 320 nm (color value (320 nm)) is attributable to the absorbance of a polyphenol such as chlorogenic acid. Therefore, individuals with a high anthocyanin content and/or a high polyphenol content in the tuberous root, which satisfy the object of the present invention, were selected using the values (color value (530 nm), color value (320 nm)) analyzed/determined by using a spectrophotometer from among the plurality of hybrid individuals obtained above.

The new variety produced in Example 1 was named "*Kyushu* No.180," and the following tests were performed (Examples 2 to 11).

Example 2: Evaluation of Characteristics of New Variety (*Kyushu* No.180) (1)

The following characteristics were measured using the tuberous root (colored sweet potato) of the new variety (*Kyushu* No.180) produced in Example 1, and the measured values were compared with those of the existing varieties (*Akemurasaki, Ayamurasaki, Murasakimasari*).

(a) Absorbance at a wavelength of 530 nm (color value (530 nm)/g)
The color value (530 nm) per gram of wet weight of colored sweet potato (color value (530 nm)/g) corresponds to the content of the purple pigment (anthocyanin pigment or the like) contained in the colored sweet potato. More specifically, a higher color value (530 nm)/g means a larger purple pigment content.
(b) Absorbance ratio between the absorbance at a wavelength of 530 nm and the absorbance at a wavelength of 320 nm, per gram of wet weight of colored sweet potato (absorbance ratio (320 nm/530 nm)/g)

Further, the absorbance at a wavelength of 530 nm is attributable to the absorbance of purple pigment (anthocyanin pigment or the like), and the absorbance at a wavelength of 320 nm is attributable to the absorbance of a polyphenol such as chlorogenic acid. Based on this, the absorbance ratio (320 nm/530 nm) refers to polyphenol content per unit pigment; in other words, the absorbance ratio (320 nm/530 nm) refers to antioxidant performance per unit pigment.

(1) Test method

Method for Finding (a) Color Value (530 nm)/g and (b) Absorbance Ratio (320 nm/530 nm)/g

About 50g of tuberous root of the new variety (*Kyushu* No.180) was cut into 5 mm squares and weighed (weighed amount $\alpha$g); thereafter, 0.5% sulfuric acid aqueous solution was added thereto so that the entire solid-liquid amount was about 250g (the entire solid-liquid amount $\beta$g). The mixture was subjected to immersion extraction for a day at room temperature (25$\pm$5°C), followed by filtration using a filter paper (ADVANTEC NO.5C), thereby obtaining a pigment liquid extract.

The prepared pigment liquid extract was appropriately diluted (y-fold) with McIlvaine's buffer (pH 3.0), and absorbance $A_{530}$ at the maximum absorption wavelength around 530 nm, absorbance $A_{320}$ at the maximum absorption wavelength around 320 nm, and absorbance $A_{700}$ at the maximum absorption wavelength around 700 nm were measured using a V-560 (JASCO Corporation) ultraviolet and visible spectrophotometer. The color value (530 nm) per gram of wet weight of sweet potato tuberous root (color value (530 nm)/g), and absorbance ratio (320nm/530nm) per gram of wet weight of colored sweet potato (absorbance ratio (320nm/530nm)/g) were calculated according to the following calculating formulas. Further, (c) (color value (530 nm)/g) $\times$ (absorbance ratio (320nm/530nm)/g) was calculated by multiplying (a) by (b).

(a) Color Value (530 nm) per Gram of Wet Weight of Colored Sweet Potato

$$\text{Color Value (530nm)/g} = (A_{530}-A_{700}) \times \gamma \times \beta \div \alpha \div 10$$

(b) Absorbance Ratio 320 nm/530 nm per Gram of Wet Weight of Colored Sweet Potato

$$\text{Absorbance Ratio (320nm/530 nm)/g} = (A_{320} - A_{700}) \div (A_{530} - A_{700})$$

(2) Test results

Table 5 shows the results.

Table 5

| Variety Line | Color Value (530nm) per Wet Weight of Colored Sweet Potato (Color Value(530nm) /g) | Absorption Ratio (320nm/530nm) per Wet Weight of Colored Sweet Potato (Absorption Ratio (320nm/530nm)/g) | Color Value (530nm)/g ×Absorption Ratio (320nm/530nm)/g |
|---|---|---|---|
| *Kyushu* No.180 | 25.82 | 2.26 | 58.35 |
| *Ayamurasaki* | 9.45 | 2.45 | 23.15 |
| *Murasakimasari* | 10.45 | 2.59 | 27.07 |
| *Akemurasaki* | 11.31 | 2.15 | 24.32 |

The results of Table 5 revealed that *Kyushu* No.180 had a significantly high color value (530 nm) per gram of wet weight of colored sweet potato (color value (530 nm)/g), compared with the existing colored sweet potato. The results also revealed that the absorbance ratio (320 nm/530 nm) per gram of wet weight of the colored sweet potato (absorbance ratio (320 nm/530 nm)/g) was substantially the same as that of the existing colored sweet potato.

This revealed that *Kyushu* No.180 has a significantly high content of purple pigment (anthocyanin pigment, or the like). Further, since *Kyushu* No.180 has, as with the existing colored sweet potato, high antioxidant performance per unit pigment (absorbance ratio (320 nm/530 nm)) despite the large content of the purple pigment, *Kyushu* No.180 was assumed to stably contain a high content of purple pigment, and therefore be useful as a colored sweet potato as a pigment material.

Example 3: Evaluation of Characteristics of New Variety *(Kyushu* No.180) (2)

The aroma component analysis was performed by the method shown below using the tuberous root (colored sweet potato) of the new variety *(Kyushu* No.180) produced in Example 1, and the measured results were compared with those of the existing varieties (*Ayamurasaki, Murasakimasari, Akemurasaki*).

(1) Test method

(1-1) Measurement of Aroma Component Content

100 mL of dichloromethane was added to 100 g of the pigment extract (pigment liquid extract) obtained in Example 2, followed by liquid-liquid extraction at room temperature; thereafter, the organic layer was isolated. The organic layer was dehydrated using anhydrous sodium sulfate, and the extract obtained by concentration under reduced pressure using an evaporator was used as a GC/MS measurement sample.

The GC/MS analysis conditions are as follows.

- GC/MS Analysis Conditions

Gas Chromatograph (GC): Agilent 6890N (Agilent Technologies, Inc.)
Mass Selective Detector (MSD): Agilent 5975 (Agilent Technologies, Inc.)
Column: Agilent J&W DB-WAX (60 m × 0.25 mm) (Agilent Technologies, Inc.)
Oven Temperature: 50°C(2 min)→220°C(3°C/min.)

(1-2) Analysis of Principal Component of Aroma Component

A principal component analysis (PCA) was performed using the peak area percentages of 23 aroma components in total obtained by GC/MS analysis with statistical analysis software (JMPver.10: SAS Institute Inc.).

(2) Test results

(2-1) Aroma Component Content

Fig. 2 shows the results of GC/MS analysis.
Twenty-three aroma components (volatile components) were obtained by GC/MS analysis.
Fig. 3 (lower left) shows the results of a comparison of the total amount of these aroma components of the new variety (*Kyushu* No.180) with those of the existing varieties (*Ayamurasaki, Murasakimasari, Akemurasaki*) (as a relative ratio based on the total amount (=100) of the aroma components of the new variety) .
Fig. 3 (lower right) also shows the results obtained by preparing a pigment extract for each variety according to the method of Example 2, finding the color value (530 nm) of the pigment extract, and converting the total amount of aroma components to a value per color value (530 nm).
As shown in the table in Fig. 3, the color value (530 nm) (= 5.2) of the pigment extract (pigment-containing composition) prepared from the new variety was at least two times higher (1.9-2.3) than those of the existing varieties. Thus, it was confirmed that the colored sweet potato itself contains a large amount of pigment component.
Further, although the total amount of aroma components contained in the new variety is not significantly high, because the color value (530 nm) is high as shown above, when the total amount of aroma components is converted to a value per color value (530 nm), the resulting value (converted value per color value (530 nm) = 19) is significantly lower than the values (44 to 125) of the existing varieties, and even less than 1/2 of the value (44) of *Ayamurasaki* having the lowest value among the existing varieties.
The results thus revealed that the new variety *(Kyushu* No.180) has a relatively low content of aroma component, despite the large amount of pigment component; therefore, it was confirmed that the new variety is useful as a pigment material for efficiently obtaining a pigment with smaller aroma.

(2-2) Analysis of Principal Component of Aroma Component

Fig. 4 shows the results of principal component analysis (PCA).
The characteristic components of the new variety are zingerone, acetoin, isoamyl alcohol, and isobutyric acid.
As shown in Fig. 4, the characteristic components of the new variety (*Kyushu* No.180) obtained by principal component analysis (PCA) were different from those of the existing varieties (*Ayamurasaki, Murasakimasari,* and *Akemurasaki*).

Example 4-1: Evaluation of Characteristics of New Variety *(Kyushu* No.180) (3)

Test samples of the tuberous roots (extracts obtained by extraction with 0.5% sulfuric acid aqueous solution) of the varieties obtained in Example 2 (new variety: *Kyushu* No.180; existing varieties: *Ayamurasaki, Murasakimasari, Akemurasaki*) were subjected to HPLC, and the pigment components (including anthocyanin derived from colored sweet potato) contained in these varieties (tuberous roots) were analyzed.

(1) Test Method

Each pigment extract prepared in Example 2 was diluted with 1% formic acid aqueous solution so that the color value (530 nm) is 1, and the resulting diluent was used as the HPLC test sample and subjected to HPLC under the following conditions.

Conditions of HPLC

HPLC Apparatus: JASCO LC-2000Plus series (JASCO Corporation)
Column: Develosil (Registered Trademark) C30-UG-5(ϕ4.6×250nm) (Nomura Chemical Co., Ltd.)
Column temperature: 40°C
Mobile phase: (a) 1v/v% formic acid aqueous solution, (b) acetonitrile
Gradient conditions: 0→15 minutes, (a) 95%→82%, (b) 5%→18% 15→45 minutes, (a) 82%→30%, (b) 18%→70% 45→55 minutes, (a) 30%→20%, (b) 70%→80% 55→60 minutes, (a) 20%→0%, (b) 80%→100%
Flow Rate: 1.0 mL/min
Sample injection amount: 20 μL
Detection: Photodiode array detector (530nm, 320nm).
The HPLC profile (profile of pigment component) of the new variety detected at a wavelength of 530 nm was compared with the profiles of the reference standards (YGM-2, YGM-1a, YGM-1b, YGM-3, YGM-4b, YGM-5a, YGM-5b, YGM-6, etc.), thereby identifying the peak components; and also compared with the HPLC profiles of the existing varieties (*Ayamurasaki, Murasakimasari,* and *Akemurasaki*).

Further, by performing a detection at a wavelength of 320 nm, HPLC profile of ultraviolet component was produced, and a comparison between the new variety and the existing varieties was performed in the same manner as above.

(2) Test results

(2-1) Fig. 5 shows HPLC profiles of the varieties detected at a wavelength of 530 nm. Fig. 5A shows peak retention time and peak area detected in each variety, and Fig. 5B shows relative ratios (%) of the respective peak areas based on the total peak area (= 100%). In each figure, "Others" represents components that do not correspond to any of YGM-2, YGM-1a, YGM-1b, YGM-3, YGM-4b, YGM-5a, YGM-5b, or YGM-6; and shows the sum of the peak areas (Fig. 5A), and the sum of relative ratios (%) with regard to the sum of the peak areas based on the total peak area (= 100%) detected at a wavelength of 530 nm (Fig. 5B).

The results showed that the peaks of pigment component confirmed most in the new variety (*Kyushu* No.180) among the respective varieties are detected at a retention time of 15.61 minutes and 17.52 minutes. It was also confirmed that the pigment component detected at a retention time of 15.61 minutes is p-hydroxybenzoylated (cyanidin 3-sophoroside-5-glucoside)(YGM-Oc), and that the pigment component detected at a retention time of 17.52 minutes is p-hydroxybenzoylated (peonidin 3-sophoroside-5-glucoside)(YGM-Oe). Fig. 6 shows the results of a comparison of these peak areas (relative ratio %) between the respective varieties.

Further, the relative ratios of the following two components were calculated, focusing on the contents (peak areas) of the pigment components (YGM-0e, YGM-4b, YGM-5a, YGM-6, and YGM-2).

Relative Ratio

a/b
c/b
c/d
e/a

a: peak area of pigment YGM-0e detected at a retention time of about 17.52 minutes
b: peak area of pigment YGM-4b detected at a retention time of about 22.07 minutes
c: peak area of pigment YGM-5a detected at a retention time of about 22.23 minutes
d: peak area of pigment YGM-6 detected at a retention time of about 22.84 minutes
e: peak area of pigment YGM-2 detected at a retention time of about 20.61 minutes

Figs. 7A to 7D, and Table 6 show the comparison of the results of the respective varieties. It was confirmed that all of the relative ratios of two components a/b, c/b, and c/d were the highest in the new variety (*Kyushu* No.180) among the respective varieties, and that the relative ratio e/a was the lowest in the new variety (*Kyushu* No.180) among the respective varieties.

Table 6

| | *Ayamurasaki* | *Murasakimasari* | *Akemurasaki* | *Kyushu* No.180 |
|---|---|---|---|---|
| a/b | 0.06 | 0.11 | 0.21 | 0.31 |
| c/b | 0.72 | 2.29 | 1.78 | 3.26 |
| c/d | 0.42 | 0.91 | 0.80 | 1.36 |
| e/a | 12.18 | 6.29 | 15.06 | 2.95 |

(2-2) Fig. 8 shows HPLC profile of ultraviolet component detected at a wavelength of 320 nm for each variety. Fig. 8A shows peak retention time and peak area detected in each variety, and Fig. 8B shows values obtained by dividing the respective peak areas by the total peak area detected at a wavelength of 530 nm.

The results revealed that the peaks of ultraviolet component confirmed most in the new variety (*Kyushu* No.180) among the respective varieties were the peaks detected at a retention time of about 10.92 minutes and about 24.44 minutes; in contrast, the peak of ultraviolet component confirmed least in the new variety (*Kyushu* No.180) was the peak detected at a retention time of 24.69 minutes. Fig. 9 shows the results of comparison of those peak areas (values obtained by dividing the respective peak areas by the total peak area detected at a wavelength of 530 nm) between the respective varieties.

Example 4-2: Evaluation of Characteristics of New Variety (*Kyushu* No.180) (4)

The pigment components (including anthocyanin derived from colored sweet potato) contained in the tuberous root (colored sweet potato) of the new variety (*Kyushu* No.180) harvested in 2016 were analyzed in the same manner as in Example 4-1. As a comparison, the pigment components contained in the tuberous root (colored sweet potato) of the existing varieties (*Ayamurasaki, Akemurasaki*) harvested in 2015, and the tuberous root (colored sweet potato) of the existing variety (*Ayamurasaki*) harvested in 2016 were also analyzed in the same manner. The relative ratios of two components (a/b, c/b, c/d, e/a) were calculated from the results, focusing on the contents (peak areas) of the pigment components (YGM-0e, YGM-4b, YGM-5a, YGM-6, and YGM-2). Table 7 shows the results.

Table 7

| | *Kyushu* No.180 | *Ayamurasaki* | | *Akemurasaki* |
|---|---|---|---|---|
| | Harvested in 2016 | Harvested in 2016 | Harvested in 2015 | Harvested in 2015 |
| a/b | 0.38 | 0.08 | 0.07 | 0.20 |
| c/b | 2.66 | 0.70 | 0.63 | 1.53 |
| c/d | 1.27 | 0.39 | 0.47 | 1.00 |
| e/a | 1.32 | 8.03 | 9.06 | 13.15 |

Example 5: Determination of Gene of New Variety *(Kyushu* No.180)

Regarding the retrotransposon Rtsp-1 of sweet potato, the genomic insertion site greatly varies between the individual varieties of sweet potato according to the studies of Okayama University etc. There are reports that the difference serves as an index of screening of sweet potato based on the variety, or as an index of determining the variety of the raw material of a sweet potato processed product (e.g. "Determination of Variety of Material of Sweet Potato Processed Product using Retrotransposon," Natsuko Ooe et al., Breeding Research 6, 169-177, (2004); "Study of Quality Improvement of Steamed and Dried Sweet Potato Slices," Toshikazu Kuranouch et al., Journal of Crop Research (Bull. Natl. Inst. Crop Sci.) 11, 49-65, (March 2010)).

Therefore, retrotransposon Rtsp-1 insertion site was searched for 27 kinds of colored sweet potatoes (the new variety, the existing varieties, and parent strains of these varieties (including crossing parents)) (see the tables of Figs. 10 and 11) with respect to genomic DNA extracted from the plants. The results revealed that there are two Rtsp-1 insertion sites (CL1836, CL1056) inherent in the new variety (*Kyushu* No.180) and the mother strain (*Kyukei* 04208-2) thereof.

(1) Test Method

(1-1) DNA Extraction

The genomic DNA for each variety (plant) of sweet potato was extracted from 100 mg of fresh leaves based on the CTAB (cetyltrimethylammonium bromide) method (Tobacco DNA/RNA isolation method, Takahiko Hayakawa (1997), "New Edition of Plant PCR Experiment Protocol" pp. 49-56, Isao Shimamoto, supervised by Takuji Sasaki, Shujunsha, Tokyo).

More specifically, DNA of each variety of sweet potatoes was extracted from 30 to 50 mg of non-expanded leaf obtained from the seedbed using a DNeasy Plant Mini Kit (QIAGEN).

(1-2) PCR Amplification of Rtsp-1 Insertion Site

PCR for the amplification of a region having the Rtsp-1 insertion sites (CL1836 and CL1056) was performed using the 27 kinds of sweet potato and the primers shown in Table 8.

More specifically, the amplification of the Rtsp-1 insertion site by PCR was performed using a 10μL reaction fluid containing 1×GoTaq Colorless Master Mix (Promega Corporation), 4pmol Rtsp-1_ppt primer (hereinafter simply referred to as ppt primer"), 4pmol insertion site primer (reverse primer: CL1836 primer, CL1056 primer), and 20ng DNA (temperature condition: 2 minutes at 94°C → [30 seconds at 94°C, 30 seconds at 58°C, 1 minute at 72°C] × 30 times → 5 minutes at 72°C) .

Further, in order to confirm the quality of the extracted DNA, amplification was performed under the same conditions using type II starch-synthesizing enzyme gene (SSII) primer. The electrophoresis of the PCR-amplified product was performed using a microchip electrophoresis device (MultiNA: Shimadzu Corporation).

Table 8

| Primer Name | Base Sequence of Primer | SEQ ID NO | Amplified Fragment Length (Expectation Value: bp) |
|---|---|---|---|
| Forward: Ppt Primer | 5'-ATCTAATCTTCAAGTGGGAGATTGTCG-3' | 1 | - |
| Reverse: CL1836 Primer | 5'-GGTCCAATGCAAGTAAGGTATACAACTTAAACCTCTTATGTCTATGAAGT-3' | 2 | 522bp |
| Reverse: CL1056 Primer | 3'-GAAACACTTGATGTGAACTCCACAACATGATGAGAATTACTTGTGGCAAC-5' | 3 | 512bp |

(2) Test Results

Fig. 10 shows the results of PCR using ppt primer (SEQ ID NO:1) as the forward primer, and CL1836 primer (SEQ ID NO:2) as the reverse primer; and Fig. 11 shows the results of PCR using ppt primer (SEQ ID NO:1) as the forward primer, and CL1056 primer (SEQ ID NO:3) as the reverse primer. In each figure, the 209bp band is a positive control for confirming that the PCR reactions were securely performed.

As shown in Figs. 10 and 11, it was confirmed that the Rtsp-1 insertion sites (CL1836 and CL1056) are not present in any existing varieties of colored sweet potatoes or their crossing parents, and are present only in the new variety (*Kyushu* No.180) of the present invention and the mother strain thereof (a crossing parent: 04208-2).

Example 6: Preparation of Colored Sweet Potato Pigment

A colored sweet potato was cut by a food processor, and immersed in a sulfuric acid aqueous solution (3-fold in volume relative to the colored sweet potato) having a concentration of 0.3 wt%. Subsequently, the mixture was stirred for an hour with a stirrer at room temperature; and was allowed to stand overnight, thereby extracting a pigment component from the colored sweet potato.

Thereafter, the extract was isolated in a gauze, and subjected to solid-liquid separation by being squeezed in the gauze. The collected liquid extract was made to pass through a 50-mesh sieve; and the resulting pigment liquid extract was heated and then cooled to 40°C, and then allowed to stand overnight. The supernatant (about 1/2) and the precipitate (about 1/2) were separated, and the precipitate was further subjected to centrifugation (10000 rpm, 5 minutes) to collect a supernatant. The obtained supernatant was mixed with the supernatant collected prior.

75 g of a filtration adjuvant (Radiolite #700: Showa Chemical Industry Co., Ltd.) was added as a body feed to 10000 mL of the supernatant obtained above, and suction filtration was performed using 25 g of the same filtration adjuvant as a precoat with a filter paper (ADVANTEC No. 2, $\phi$12.5cm).

Among the filtrate collected by the suction filtration, a portion corresponding to 20000 color quantity was weighed, and the following resin treatment was performed.

- Resin Treatment

By allowing contact with a Diaion HP-20 synthetic adsorbent resin (resin amount = 500 mL, SV=3.0$\pm$0.2, Mitsubishi Chemical Corp.), the pigment component was made to adsorb thereto, and the resin was washed well with 1500 mL of water (SV=1.0$\pm$0.2), followed by desorption elution with 500 mL of a 50% ethanol aqueous solution (SV=0.5$\pm$0.1). Subsequently, by allowing contact with an Amberlite FPC3500 ion-exchange resin (resin amount = 100 mL, SV1.5$\pm$0.2, Organo Corporation), the contaminants were made to adsorb thereto, and the liquid passed through the resin was collected as a resin treatment solution.

The solution obtained by the resin treatment was subjected to suction filtration using a filter paper (ADVANTEC No.5, $\phi$12.5cm) and the collected filtrate was concentrated so that the color value (530 nm) was 300 or more. Subsequently, the concentrated liquid was adjusted so that the color value (530 nm) was 85 using a 95 volume% ethanol aqueous solution, and the ethanol concentration was 20 volume%; followed by sterilization, thereby obtaining a colored sweet potato pigment (pigment composition: purified matter).

Example 7: Characteristics of Colored Sweet Potato Pigment Derived From New Variety (*Kyushu* No.180) (1)

The color value and aroma component content of the colored sweet potato pigment (colored potato pigment) prepared in Example 6 were measured by the following method. The color value and aroma component content of colored potato pigments prepared from the existing colored sweet potato varieties *Ayamurasaki* and *Akemurasaki* were also measured in the same manner for comparison.

(1) Measurement of Color Value

The color value was measured using, as the test sample, the colored sweet potato pigment (pigment composition) prepared in Example 6.

The pigment composition was appropriately diluted (y-fold) with McIlvaine's buffer (pH 3.0), and absorbance $A_{530}$ at the maximum absorption wavelength around 530 nm was measured using a V-560 (JASCO Corporation) ultraviolet and visible spectrophotometer. The color value (530 nm) was calculated according to the following calculating formula.

$$\text{Color value (530nm)} = A_{530} \times \gamma \div 10$$

(2) Measurement of Aroma Component Content

10 μg of 3-heptanol was added as an internal standard substance to a solution obtained by diluting 10 g of colored sweet potato pigment with 200 mL of ion-exchanged water. 200 mL of dichloromethane was added thereto, followed by liquid-liquid extraction at room temperature; thereafter, the organic layer was isolated. The organic layer was dehydrated using anhydrous sodium sulfate, and the extract obtained by concentration under reduced pressure using an evaporator was used as a GC/MS measurement sample.
The GC/MS analysis conditions are as follows.

- GC/MS analysis conditions

Gas Chromatograph (GC): Agilent 6890N (Agilent Technologies, Inc.)
MSD: Agilent 5975 (Agilent Technologies, Inc.)
Column: Agilent J&W DB-WAX (60 m × 0.25 mm) (Agilent Technologies, Inc.)
Oven Temperature: 50°C(2 min)→220°C(3°C/min.)

(3) Test Results

(3-1) Aroma Component Content

Fig. 12 shows the results of GC/MS analysis.
Thirty-four aroma components (volatile components) were confirmed by GC/MS analysis (Fig. 12).
The total amount of aroma components was determined, and the total amount of aroma components per gram of colored sweet potato pigment (concentration: μg/g) was calculated. Table 9 shows the results of a comparison of the value of the new variety with the values of the existing varieties (relative ratio based on the total amount (=100) of the aroma components of the new variety). Table 9 also shows the results obtained by determining, for each colored sweet potato pigment, the color value (530 nm) of the colored sweet potato pigment (pigment composition) prepared in Example 6; and converting the total amount of aroma components to a value per color value (530 nm).

Table 9

| | *Ayamurasaki* | *Akemurasaki* | *Kyushu* No.180 |
|---|---|---|---|
| Total Amount of Aroma Components (34 Components) (μg/g) | 201.0 | 179.4 | 63.6 |
| Color Value (530nm) | 88.3 | 88.9 | 91.2 |
| Total Amount of Aroma Components (34 Components) (μg/g) per Color Value (530nm) | 2.28 | 2.02 | 0.70 |
| Total Amount of Aroma Components (Concentration) (μg/g) when Color Value $E^{10\%}_{1cm}$ at 530nm is 80 | 182.1 | 161.4 | 55.8 |
| Relative Ratio based on New Type (=1). | 3.26 | 2.89 | 1 |
| The total amount of aroma components (34 components) is an approximate value calculated based on the peak areas of 34 components (on the condition that the response factor with respect to the internal standard =1). | | | |

As shown in Table 9, it was confirmed that the total amount of aroma components (34 components) contained in the colored sweet potato pigment prepared from the new variety was lower than those prepared from the existing varieties (*Ayamurasaki, Akemurasaki*). It was confirmed that the total amount of aroma components per color value (530 nm), which was 0.70, was significantly lower than the values of the pigments derived from the existing varieties.
The results confirmed that the pigment composition prepared from the new variety (*Kyushu* No.180) is useful as a pigment preparation for use in foods and beverages, etc. because of the small content of aroma components per color value (530 nm) .

Example 8: Characteristics of Colored Potato Pigment Derived From New Variety (*Kyushu* No.180) (2)

Test samples (pigment compositions adjusted to have a color value of 85) of the colored sweet potato pigments obtained in Example 6 (new variety: *Kyushu* No.180; existing varieties *Ayamurasaki, Akemurasaki*) were subjected to HPLC, and the pigment components (including anthocyanin derived from colored sweet potato) contained in these varieties were analyzed.

(1) Test Method

The test samples prepared in Example 6 were diluted with 1% formic acid aqueous solution so that the color value (530 nm) was 1, and the resulting diluents were used as the HPLC test samples and subjected to HPLC under the following conditions.

- Conditions of HPLC

HPLC Apparatus: JASCO LC-2000Plus series (JASCO Corporation)
Column: Develosil C30-UG-5 (ϕ4.6×250nm) (Nomura Chemical Co., Ltd.)
Column temperature: 40°C
Mobile phase: (a) 1%v/v formic acid aqueous solution, (b) acetonitrile
Gradient conditions: 0→15 minutes, (a) 95%→82%, (b) 5%→18% 15→45 minutes, (a) 82%→30%, (b) 18%→70% 45→55 minutes, (a) 30%→20%, (b) 70%→80% 55→60 minutes, (a) 20%→0%, (b) 80%→100%
Flow Rate: 1.0 mL/min
Sample Injection Amount: 20 μL
Detection: Photodiode array detector (530 nm, 320nm).

The HPLC profile (profile of pigment component) detected at a wavelength of 530 nm was compared with the profiles of the reference standards (YGM-2, YGM-1a, YGM-1b, YGM-3, YGM-4b, YGM-5a, YGM-5b, YGM-6, etc.), thereby identifying the peak components, and also compared with the HPLC profiles of the existing varieties.

Further, by performing a detection at a wavelength of 320 nm, HPLC profile of ultraviolet component was produced, and a comparison between the new variety and the existing varieties was performed in the same manner as above.

(2) Test Results

(2-1) Fig. 13 shows HPLC profiles of the varieties detected at a wavelength of 530 nm. Fig. 13A shows peak retention time and peak area detected in each variety, and Fig. 13B shows relative ratios (%) of the respective peak areas based on the total peak area (= 100%).

The results showed that the peaks of pigment component confirmed most in the pigment derived from the new variety (*Kyushu* No.180) among the respective varieties are detected at a retention time of 17.52 minutes and 22.23 minutes. It was confirmed that the pigment component detected at a retention time of 17.52 minutes is p-hydroxybenzoylated (peonidin 3-sophoroside-5-glucoside) (YGM-0e). Further, the pigment component detected at a retention time of 22.23 minutes is YGM-5a.

The relative ratios of two components, i.e., a/b (YGM-0e/YGM-4b), c/b (YGM-5a/YGM-4b), c/d (YGM-5a/YGM-6), and e/a (YGM-2/YGM-0e), were calculated in the same manner as in Example 4, focusing on the contents (peak areas) of the pigment components (YGM-0e, YGM-4b, YGM-5a, YGM-6, and YGM-2). Table 10 and Figs. 14A to 14D show the results of a comparison between these pigments. It was confirmed that, among these relative ratios of two components, a/b, c/b, and c/d were the highest in the pigment derived from the new variety among the respective varieties, and the relative ratio e/a was the lowest in the pigment derived from the new variety among the respective varieties.

Table 10

| | *Ayamurasaki* | *Akemurasaki* | *Kyushu* No.180 |
|---|---|---|---|
| a/b | 0.09 | 0.28 | 0.60 |
| c/b | 0.79 | 1.81 | 3.19 |
| c/d | 0.63 | 1.14 | 1.96 |
| e/a | 8.47 | 9.85 | 1.29 |

(2-2) Fig. 15 shows HPLC profile of ultraviolet component detected at a wavelength of 320 nm with respect to the respective varieties. Fig. 15A shows peak retention time and peak area detected in the respective pigments, and Fig. 15B shows values obtained by dividing the peak areas by the total peak area detected at a wavelength of 530 nm.

The results showed that the peaks of ultraviolet component confirmed most in the pigment derived from the new variety (*Kyushu* No.180) among the respective varieties were detected at a retention time of 10.92 minutes, 24.44 minutes, and 24.69 minutes. Fig. 16 shows the results of a comparison between the respective varieties with regard to the value obtained by dividing the peak area by the total peak area detected at a wavelength of 530 nm.

Example 9: Characteristics of Colored Potato Pigment Derived From New Variety (*Kyushu* No.180) (3)

A test beverage was prepared using the pigment composition prepared in Example 6, and the pigment residual ratio and changes in color tone with respect to light and heat were evaluated. Further, for composition, a test beverage was prepared in the same manner using a pigment composition prepared from the existing variety *Ayamurasaki* in the same manner, and the pigment residual ratio and changes in color tone with respect to light and heat were measured.

(1) Preparation of Test Beverage

13.3 wt% of high-fructose corn syrup (Brix75°), 0.2 wt% of citric acid (anhydrous), 0.052 wt% of trisodium citrate, and 0.03 wt% of pigment composition (color value (530nm)=80) were added to drinking water so that the total amount was 100 wt%; and the mixture was adjusted to have Brix10° and a pH of 3.0. After filling a 200-mL capacity PET bottle with the prepared test beverage, the test beverage was hot-packed at 93°C for sterilization.

(2) Test Method

(a) Light Resistance Test

The test beverage prepared above was allowed to stand for 5 days and 10 days in an irradiator under the following conditions.
Irradiator: Cultivation Chamber CLH-301 (Tomy Seiko Co., Ltd.)
Light Source/Illuminance: White fluorescent light, 10000 lux
Irradiation Temperature: 30°C

(b) Heat Resistance Test

The test beverage prepared above was allowed to stand in the dark at 50°C for five days and ten days.
The absorbance at the maximum absorption wavelength around a wavelength of 530 nm was measured before and after the test, and the pigment residual ratio (%) was calculated according to the following formula.

$$\text{Pigment residual ratio (\%)} = (\text{absorbance after the test}/\text{absorbance before the test}) \times 100$$

Further, the color tone was evaluated using a Munsell HVC color system before and after the test, and the values were compared.

(3) Test results

Table 11 shows the results of pigment residual ratio (%), and Table 12 shows changes in color tone.

Table 11

Pigment Residual Ratio (%)

| | Fluorescent Light 10000 lux (30°C) | | Stored at 50°C | |
|---|---|---|---|---|
| | 5 Days | 10 Days | 5 Days | 10 Days |
| *Kyushu* No.180 | 51.3 | 38.9 | 71.9 | 55.6 |
| *Ayamurasaki* | 46.6 | 35.0 | 63.8 | 46.2 |

Table 12

Color Tone Change

| | Control | Fluorescent Light 10,000 lux (30°C) | | Stored at 50°C | |
|---|---|---|---|---|---|
| | | 5 Days | 10 Days | 5 Days | 10 Days |
| *Kyushu* No.180 | | | | | |
| L(Brightness:0 Black↔100 White) | 88.3 | 93.6 | 94.7 | 91.3 | 92.9 |
| a(-Green↔+Red) | 22.5 | 12.7 | 9.2 | 17.1 | 13.0 |
| b(-Blue↔+Yellow) | -3.9 | -2.3 | 1.0 | -3.1 | -1.8 |
| Hue | 7.2RP | 7.2RP | 8.2RP | 7.1RP | 7.9RP |
| Chroma | 22.9 | 12.9 | 9.2 | 17.4 | 13.1 |
| ΔE (Color Difference) | 0.0 | 11.3 | 15.1 | 6.2 | 10.8 |
| *Ayamurasaki* | | | | | |
| L(Brightness:0 Black↔100 White) | 87.8 | 93.5 | 95.0 | 91.9 | 93.7 |
| a(-Green↔+Red) | 23.2 | 11.1 | 8.3 | 15.9 | 11.2 |
| b(-Blue↔+Yellow) | -4.6 | -1.9 | 0.9 | -3.2 | -1.6 |
| Hue | 6.9RP | 7.3RP | 8.2RP | 6.8RP | 7.8RP |
| Chroma | 23.7 | 11.2 | 8.4 | 16.2 | 11.3 |
| ΔE (Color Difference) | 0.0 | 13.7 | 17.0 | 8.5 | 13.8 |

"Control" denotes measurement results of test sample before the light resistance test and the heat resistance test.

As shown above, it was confirmed that the pigment derived from *Kyushu* No.180 of the present invention is equivalent or superior to the pigment derived from the existing variety *Ayamurasaki* in terms of both light (fluorescent light) resistance and heat resistance.

Example 10: Production of a Variety of Colored Sweet Potato Having High Anthocyanin Content and Evaluation of Characteristics

A colored sweet potato was grown by cultivation under normal cultivation conditions by vegetative propagation (asexual propagation) using, as a seed potato, the new variety (*Kyushu* No.180) produced in Example 1, thereby harvesting a colored sweet potato with a high anthocyanin content having the same genetic information as that of the colored sweet potato obtained in Example 1. The characteristics of the obtained colored sweet potato (*Kyushu* No.180) with a high anthocyanin content were evaluated according to the following method.

(1) According to the method of Example 2, using a pigment liquid extract prepared from the colored sweet potato (tuberous root), (a) absorbance per gram of wet weight of colored sweet potato at the maximum wavelength around a wavelength of 530 nm (color value (530 nm)/g), and (b) absorbance ratio (320 nm/530 nm) per gram of wet weight of colored sweet potato (absorbance ratio (320 nm/530 nm)/g) were measured. Table 13 shows the results.

Table 13

| Color Value (530nm) per Wet Weight of Colored Sweet Potato (Color Value (530nm)/g) | Absorption Ratio (320nm/530nm) per Wet Weight of Colored Sweet Potato (Absorption Ratio (320nm/530nm)/g) | Color Value(530nm)/g× Absorption Ratio (320nm/530nm)/g |
|---|---|---|
| 18.69 | 2.65 | 49.52 |

As in Example 2, the resulting colored sweet potato having a high anthocyanin content has a high color value (530 nm) per gram of wet weight of colored sweet potato (color value (530 nm)/g) compared with the existing colored sweet potato; and also ensures a high antioxidant performance (absorbance ratio (320 nm/530 nm)/g) per unit pigment, as in the existing colored sweet potato. Thus, it was shown that the colored sweet potato stably contains a high content of purple pigment.

(2) According to the method of Example 4, pigment components contained in the tuberous root of the colored sweet potato were analyzed using HPLC, and relative ratios of the two components, i.e., a/b, c/b, c/d, and e/a were calculated. Fig. 17 shows HPLC profiles detected at a wavelength of 530 nm, and Table 14 shows the relative ratios of two components.

Table 14

| | |
|---|---|
| a/b | 0.70 |
| c/b | 3.46 |
| c/d | 1.23 |
| e/a | 1.95 |

(3) A concentrated liquid extract was obtained from the colored sweet potato (*Kyushu* No.180) having a high anthocyanin content obtained above according to the following method.

130 kg of colored sweet potato (*Kyushu* No.180) was pulverized using a cutter; thereafter, 0.3% sulfuric acid aqueous solution was added thereto so that the entire solid-liquid amount was 500 L. The mixture was subjected to extraction under stirring for an hour at room temperature, and the solid portion was removed by centrifugation, thereby obtaining a pigment liquid extract. The pigment liquid extract was concentrated to a color value of 20, thereby obtaining a concentrated liquid extract.

According to the method of Example 4, pigment components of the concentrated liquid extract were analyzed using HPLC, and relative ratios of the two components, i.e., a/b, c/b, c/d, and e/a, were calculated. Fig. 18 shows HPLC profiles detected at a wavelength of 530 nm, and Table 15 shows the relative ratios of two components.

Table 15

| | |
|---|---|
| a/b | 1.07 |
| c/b | 5.49 |
| c/d | 1.74 |
| e/a | 1.07 |

Example 11: Determination of Gene of New Variety (*Kyushu* No.180)

With respect to the respective varieties of colored sweet potato (new variety: *Kyushu* No.180, existing varieties: *Kyukei* 04222-50, *Kyukei* 04208-2, *Akemurasaki, Murasakimasari, Ayamurasaki*), PCR amplification was performed in the same manner as in Example 5 using genomic DNA extracted from each plant (fresh leaves) and primer sets 3 to 9 shown in Tables 2 and 3. The following reaction fluid was used in the PCR amplification.

Reaction Fluid

| | |
|---|---|
| ExTaq: | 0.1 μL |
| 10 × ExBuffer: | 1.0 μL |
| dNTPs: | 0.8 μL |
| Forward primer (ppt primer) (1 μM): | 2.0 μL |
| Each reverse primer (1 μM): | 2.0 μL |
| DNA of each colored sweet potato: | 1.0 μL |
| dH2O: | 3.1 μL |
| Total | 10.0 μL |

Further, the amplification reaction was performed under the following temperature conditions: 4 minutes at 94°C→(30 seconds at 94°C, 30 seconds at 64-65°C, 30 seconds at 72°C) × 35 times→15 minutes at 72°C. The obtained reaction fluid was subjected to a treatment using a microchip electrophoresis device (MultiNA: Shimadzu Corporation), and the production of a PCR-amplified product was confirmed. Figs. 19A to 19G show electrophoresis images of the amplified products obtained by PCR amplification using primer sets 3 to 9.

In the image, A to F lanes respectively correspond to the results of *Kyushu* No.180, *Kyukei* 04222-50, *Kyukei* 04208-2, *Akemurasaki*, *Murasakimasari,* and *Ayamurasaki,* in this order. The results confirmed that use of primer sets 3 to 9 enables determination of the new variety (*Kyushu* No.180) of the present invention by clearly discriminating it from the existing varieties.

Sequence Listing Free Text

SEQ ID NO:1 represents the base sequence of forward primer (ppt primer), SEQ ID NO:2 represents the base sequence of reverse primer (CL1836 primer), SEQ ID NO:3 represents the base sequence of reverse primer (CL1056 primer), SEQ ID NO:4 represents the base sequence of reverse primer (CL103 primer), SEQ ID NO:5 represents the base sequence of reverse primer (Pattern228 primer), SEQ ID NO:6 represents the base sequence of reverse primer (Pattern238 primer), SEQ ID NO:7 represents the base sequence of reverse primer (Pattern264 primer), SEQ ID NO:8 represents the base sequence of reverse primer (Pattern275 primer), SEQ ID NO:9 represents the base sequence of reverse primer (Pattern290 primer), and SEQ ID NO:10 represents the base sequence of reverse primer (CL121 primer).

## Claims

**1.** A colored sweet potato having the following characteristics:

(A) the color value (530 nm) per gram of wet weight of colored sweet potato (color value (530nm)/g) is not less than 15;
(B) the absorbance ratio (320 nm/530 nm) per gram of wet weight of colored sweet potato (absorbance ratio (320 nm/530 nm)/g) is not less than 1.5;
(C) color value (530nm)/g × absorbance ratio (320nm/530nm)/g = not less than 30; and
(D) LTR retrotransposon (Rtsp-1) is inserted into at least two positions of the genome sequence, and an amplified product having a fragment length of 500 to 530bp is produced when a nucleic acid amplification reaction is performed using, as a test material, a part of a plant, and at least one of primer set 1 containing a forward primer having the base sequence of SEQ ID NO:1 and a reverse primer having the base sequence of SEQ ID NO:2, and primer set 2 containing a forward primer having the base sequence of SEQ ID NO:1 and a reverse primer having the base sequence of SEQ ID NO:3.

**2.** A colored sweet potato having the following characteristics:

(A) the color value (530 nm) per gram of wet weight of colored sweet potato (color value (530nm)/g) is not less than 15;
(B) the absorbance ratio (320 nm/530 nm) per gram of wet weight of colored sweet potato (absorbance ratio (320 nm/530 nm)/g) is not less than 1.5;
(C) color value (530nm)/g × absorbance ratio (320nm/530nm)/g = not less than 30; and
(E) the total amount of aroma components per color value (530 nm) per gram of wet weight of colored sweet potato (color value (530 nm)/g) is not more than 40%, based on the total amount of aroma components per color value (530 nm)/g of the existing variety *Akemurasaki.*

**3.** The colored sweet potato according to claim 1, further having the characteristic (E) below;
(E) the total amount of aroma components per color value (530 nm) is not more than 40%, based on the total amount of aroma components per color value (530 nm) of the existing variety *Akemurasaki.*

**4.** The colored sweet potato according to any one of claims 1 to 3, wherein the colored sweet potato is derived from a mother strain (*Kyukei* 04208-2) and a father strain (*Kyukei* 04222-50), and the mother strain is a sweet potato having the characteristic (D) below;
(D) LTR retrotransposon (Rtsp-1) is inserted into at least two positions of the genome sequence, and an amplified product having a fragment length of 500 to 530bp is produced when a nucleic acid amplification reaction is performed using, as a test material, a part of a plant, and at least one of primer set 1 containing a forward primer having the base sequence of SEQ ID NO:1 and a reverse primer having the base sequence of SEQ ID NO:2, and primer set 2 containing a forward primer having the base sequence of SEQ ID NO:1 and a reverse primer having the base sequence of SEQ ID NO:3.

**5.** The colored sweet potato according to any one of claims 1 to 4, wherein the colored sweet potato is a colored sweet potato for use in a pigment material.

**6.** An extract composition of the colored sweet potato according to any one of claims 1 to 5 or a purified matter thereof.

**7.** The extract composition of the colored sweet potato or the purified matter thereof according to claim 6, which is a pigment composition.

**8.** A pigment composition derived from a colored sweet potato containing at least anthocyanin pigments of pigment YGM-0e, pigment YGM-4b, pigment YGM-5a, pigment YGM-6, and pigment YGM-2, and the content ratio of each pigment satisfies at least one of (1) to (4), as peak area ratio detected by HPLC under the following conditions:

(1) a/b: 0.3 to 2
(2) c/b: 2.3 to 10
(3) c/d: 1.2 to 5
(4) e/a: 0.1 to 4.5

a: peak area of pigment YGM-0e
b: peak area of pigment YGM-4b
c: peak area of pigment YGM-5a
d: peak area of pigment YGM-6
e: peak area of pigment YGM-2

Conditions of HPLC Analysis
ODS reverse-phase column (linking group: triacontyl group): pore size (14 nm), specific surface area (300 $m^2/g$), pore volume (1.05 mg/mL), diameter and length ($\phi 4.6 \times 250$ nm)
Column temperature: 40°C
Mobile phase: (a) 1v/v% formic acid aqueous solution, (b) acetonitrile
Gradient conditions:

0→15 minutes, (a) 95%→82%, (b) 5%→18%
15→45 minutes, (a) 82%→30%, (b) 18%→70%
45→55 minutes, (a) 30%→20%, (b) 70%→80%
55→60 minutes, (a) 20%→0%, (b) 80%→100%

Flow Rate: 1.0 mL/min
Sample injection amount: 20 μL
Detection: Photodiode array detector (530 nm).

**9.** The pigment composition according to claim 8, wherein the total amount of aroma components is not more than 120 ppm when the color value at the maximum absorption wavelength around a wavelength of 530 nm is $E^{10\%}_{1cm}$=80.

**10.** The pigment composition according to claim 8 or 9, wherein the colored sweet potato is the colored sweet potato according to any one of claims 1 to 5.

**11.** A method for determining a variety of an edible colored sweet potato, or an edible colored sweet potato contained in an edible composition comprising, as a raw material, an edible colored sweet potato, the method comprising the steps (1) and (2), or the steps (1') and (2'):

(1) a step of performing a nucleic acid amplification reaction using, as a template, DNA prepared from an edible colored sweet potato or an edible composition containing an edible colored sweet potato as a raw material, and at least one of primer set 1 containing a forward primer having the base sequence of SEQ ID NO:1 and a reverse primer having the base sequence of SEQ ID NO:2, and primer set 2 containing a forward primer having the base sequence of SEQ ID NO:1 and a reverse primer having the base sequence of SEQ ID NO:3; and
(2) a step of confirming the presence or absence of production of amplified product having a fragment length of 500 to 530bp by the nucleic acid amplification reaction; or
(1') a step of performing a nucleic acid amplification reaction using, as a template, DNA prepared from an edible colored sweet potato or an edible composition containing an edible colored sweet potato as a raw material, and at least one of primer sets 3 to 9 containing a forward primer having the base sequence of SEQ ID NO:1 and a reverse primer having the base sequence of any one of SEQ ID NOs:4 to 10; and
(2') a step of confirming the presence or absence of production of amplified product having a fragment length of 550 to 650bp by the nucleic acid amplification reaction.

**12.** The method for determining a variety of an edible colored sweet potato according to claim 11, further comprising the following step (3):
(3) a step of determining, when production of corresponding amplified product is confirmed according to the result of step (2) or (2'), that the edible colored sweet potato or the edible colored sweet potato contained in the edible composition is the colored sweet potato according to any one of claims 1 to 5.

**13.** A reagent for determining a variety of an edible colored sweet potato, comprising at least one primer set selected from the group consisting of the following (a) to (i):

(a) primer set 1 containing a forward primer having the base sequence of SEQ ID NO:1 and a reverse primer having the base sequence of SEQ ID NO:2,
(b) primer set 2 containing a forward primer having the base sequence of SEQ ID NO:1 and a reverse primer having the base sequence of SEQ ID NO:3,
(c) primer set 3 containing a forward primer having the base sequence of SEQ ID NO:1 and a reverse primer having the base sequence of SEQ ID NO:4,
(d) primer set 4 containing a forward primer having the base sequence of SEQ ID NO:1 and a reverse primer having the base sequence of SEQ ID NO:5,
(e) primer set 5 containing a forward primer having the base sequence of SEQ ID NO:1 and a reverse primer having the base sequence of SEQ ID NO:6,
(f) primer set 6 containing a forward primer having the base sequence of SEQ ID NO:1 and a reverse primer having the base sequence of SEQ ID NO:7,
(g) primer set 7 containing a forward primer having the base sequence of SEQ ID NO:1 and a reverse primer having the base sequence of SEQ ID NO:8,
(h) primer set 8 containing a forward primer having the base sequence of SEQ ID NO:1 and a reverse primer having the base sequence of SEQ ID NO:9, and
(i) primer set 9 containing a forward primer having the base sequence of SEQ ID NO:1 and a reverse primer having the base sequence of SEQ ID NO:10.

**14.** The reagent for determining a variety of an edible colored sweet potato according to claim 13, for use in the execution of the method for determining a variety of an edible colored sweet potato according to claim 11 or 12.

**15.** A kit used to determine a variety of a colored sweet potato, the kit comprising the reagent for determining a variety of an edible colored sweet potato according to claim 13 or 14.

Fig. 1

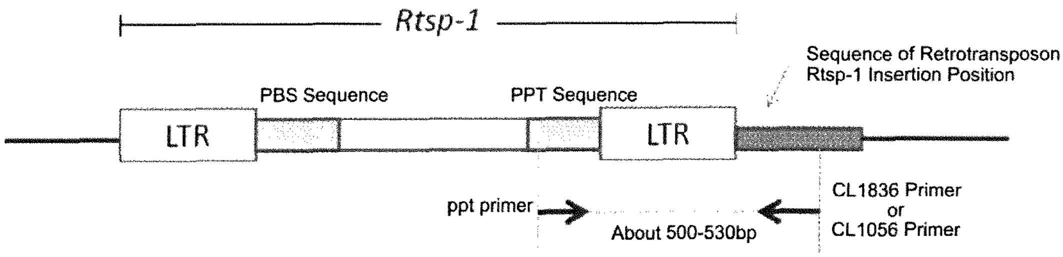
Rtsp-1
Sequence of Retrotransposon Rtsp-1 Insertion Position
PBS Sequence
PPT Sequence
LTR
LTR
ppt primer
About 500-530bp
CL1836 Primer
or
CL1056 Primer

Fig. 2

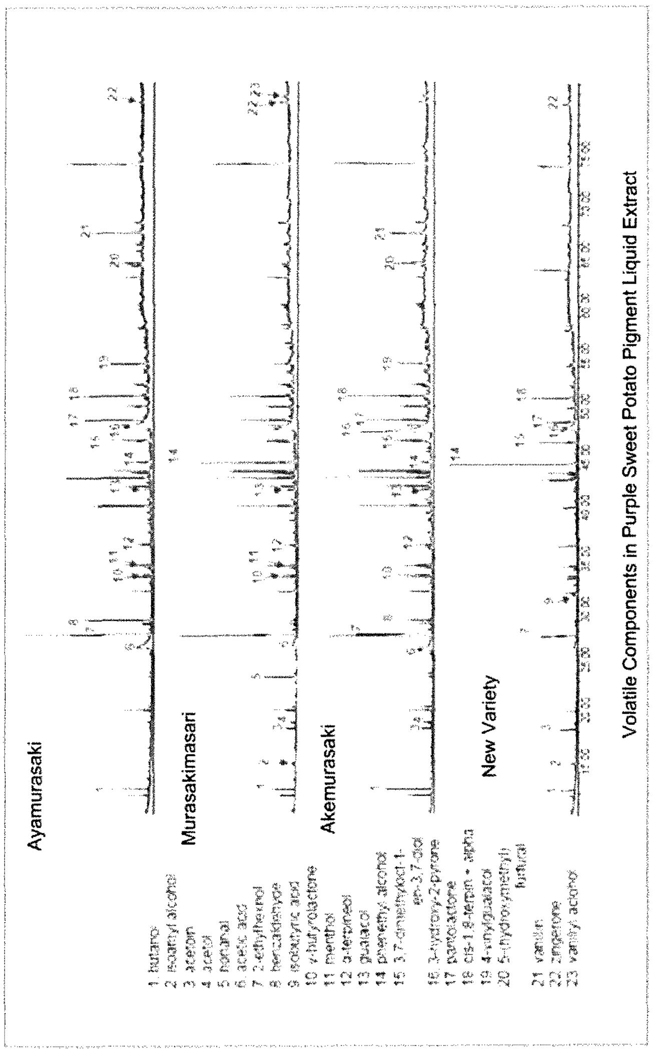
Ayamurasaki
Murasakimasari
Akemurasaki
New Variety
Volatile Components in Purple Sweet Potato Pigment Liquid Extract

Fig. 3

Relative Total Aroma Component in Purple Sweet Potato Pigment (*)

| | Ayamurasaki | Murasakimasari | Akemurasaki | New Variety |
|---|---|---|---|---|
| Color Value (530 nm) | 1.9 | 2.1 | 2.3 | 5.2 |
| Relative Amount Based on Total Aroma Component (=100)of New Variety | 84 | 139 | 288 | 100 |
| Conversion to a Value per Color Value (530 nm) | 44 | 66 | 125 | 19 |

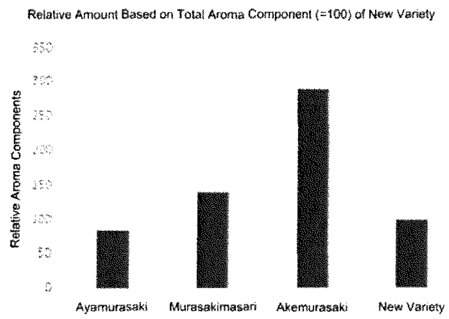


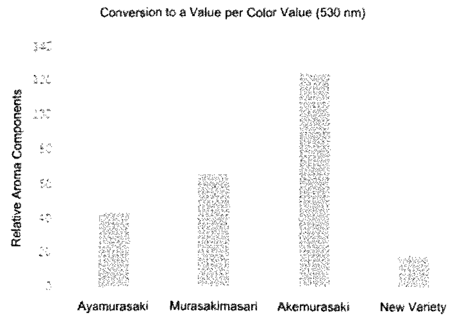


(*) Total Aroma Component was Calculated from Sum of Peak Areas of 23 Components.

Fig. 4

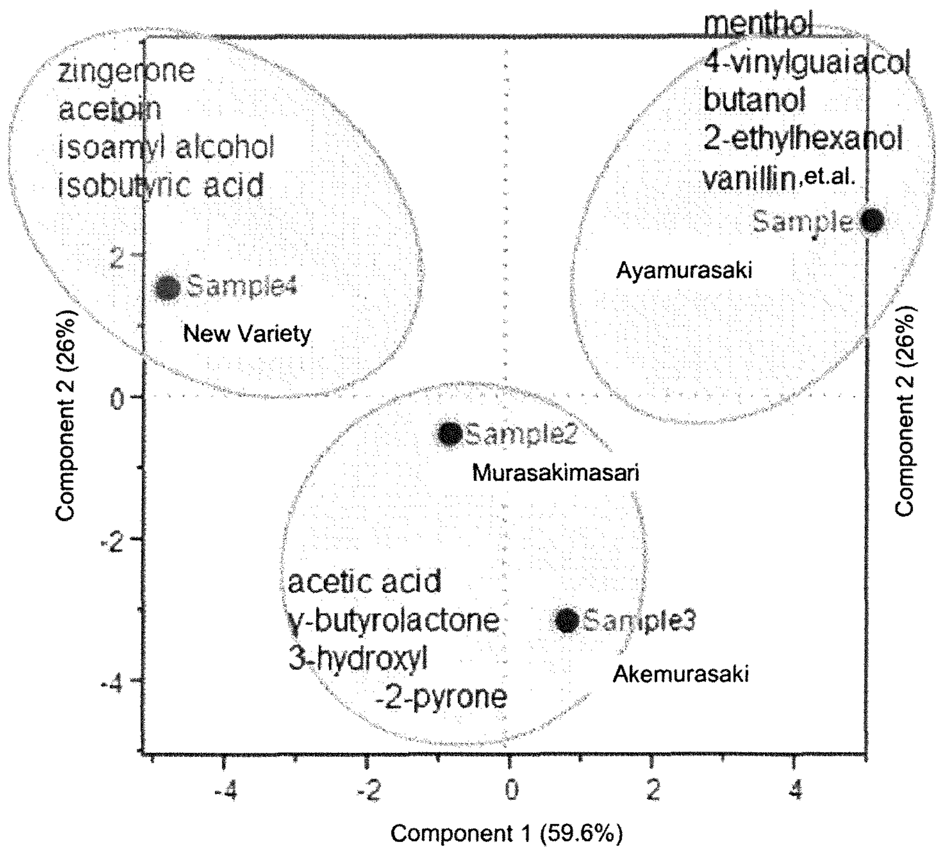

Fig. 5

A

(1) Pigment Component (Wavelength 530 nm)

| Peak Area | Aya masari | Murasaki masari | Ake masari | Kyushu No.180 | Pigment Component |
|---|---|---|---|---|---|
| R.T. | YK1 | YK3 | YK4 | YK7 | |
| 3.03 | 24134 | 23962 | 18302 | 23424 | |
| 3.21 | 56723 | 10148 | 52182 | 57207 | |
| 3.64 | 41822 | 39012 | 36951 | 41155 | |
| 11.95 | 27211 | | 124492 | 28511 | |
| 13.73 | 45225 | 32311 | 120894 | 72620 | |
| 15.61 | | | 25006 | 23536 | YGM-0c |
| 16.16 | | | 10352 | | |
| 17.52 | 19759 | 24554 | 36015 | 53215 | YGM-0e |
| 18.05 | 21860 | | 14274 | | |
| 18.53 | 21012 | | 34080 | 11873 | |
| 20.17 | 37740 | 27075 | 46850 | 32782 | |
| 20.61 | 240727 | 154456 | 542545 | 156757 | YGM-2 |
| 20.92 | 148396 | 102250 | 214134 | 155818 | YGM-1a+YGM-1b |
| 21.69 | 812575 | 832280 | 981890 | 810638 | YGM-3+YGM-5b |
| 22.07 | 346779 | 219674 | 171061 | 173101 | YGM-4b |
| 22.23 | 250842 | 503525 | 304084 | 564566 | YGM-5a |
| 22.84 | 593428 | 551290 | 381494 | 415824 | YGM-6 |
| 23.15 | | 13722 | | | |
| 23.39 | 35893 | 15334 | | | |
| 23.59 | | 10899 | | | |
| 24.04 | 32801 | 18372 | | | |
| Others | 364179 | 215490 | 518407 | 344333 | |
| Total Peak Area | 2756826 | 2578975 | 3114615 | 2619038 | |

B

| Peak Area /Total Peak Area | Aya masari | Murasaki masari | Ake masari | Kyushu No.180 |
|---|---|---|---|---|
| R.T. | YK1 | YK3 | YK4 | YK7 |
| 3.03 | 1% | 1% | 1% | 1% |
| 3.21 | 2% | 0% | 2% | 2% |
| 3.64 | 2% | 2% | 1% | 2% |
| 11.95 | 1% | 0% | 4% | 1% |
| 13.73 | 2% | 1% | 4% | 3% |
| 15.61 | 0.0% | 0.0% | 0.8% | 0.9% |
| 16.16 | 0% | 0% | 0% | 0% |
| 17.52 | 0.72% | 0.95% | 1.16% | 2.00% |
| 18.05 | 1% | 0% | 0% | 0% |
| 18.53 | 1% | 0% | 1% | 0% |
| 20.17 | 1% | 1% | 2% | 1% |
| 20.61 | 9% | 6% | 17% | 6% |
| 20.92 | 5% | 4% | 7% | 6% |
| 21.69 | 29% | 32% | 32% | 31% |
| 22.07 | 13% | 9% | 5% | 7% |
| 22.23 | 9% | 20% | 10% | 22% |
| 22.84 | 22% | 21% | 12% | 16% |
| 23.15 | 0% | 1% | 0% | 0% |
| 23.39 | 1% | 1% | 0% | 0% |
| 23.59 | 0% | 0% | 0% | 0% |
| 24.04 | 1% | 1% | 0% | 0% |
| Others | 13% | 8% | 17% | 13% |

Components Other Than YGM2, 1a, 1b, 3, 5b, 4b, 5a, and 6

Fig. 6

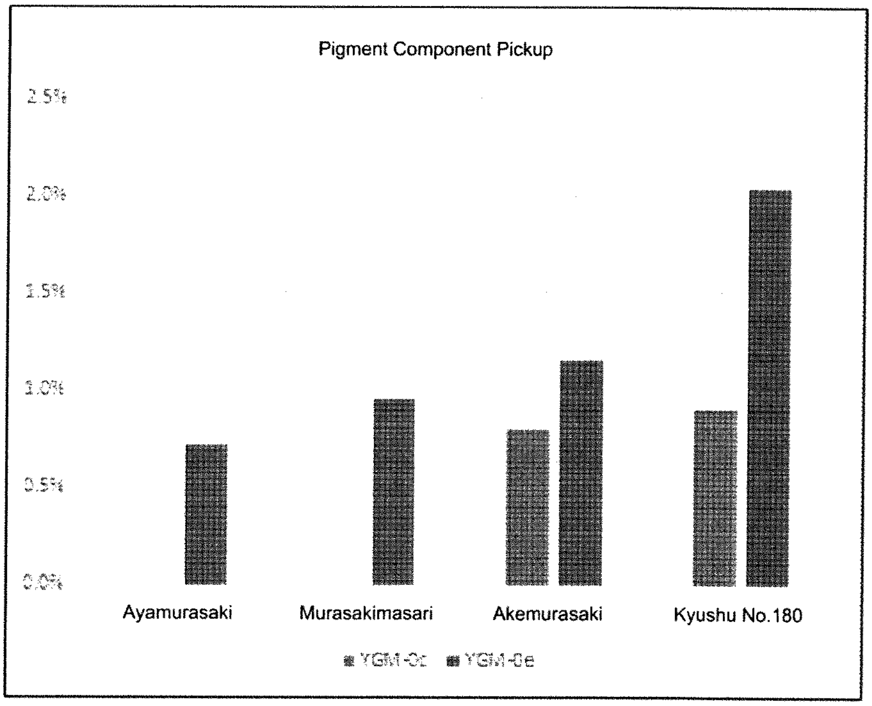

Fig. 7A

A

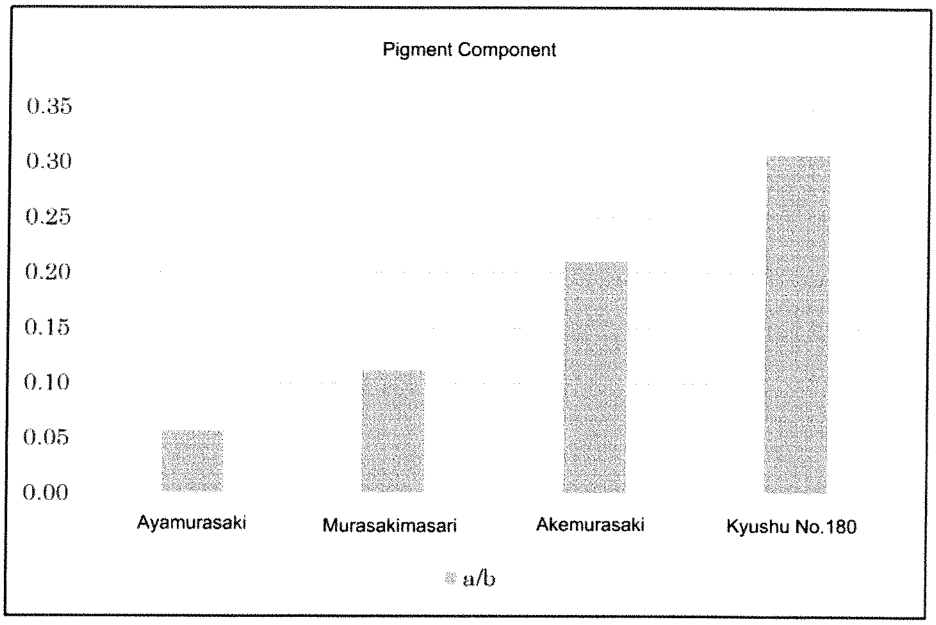


Fig. 7B

B

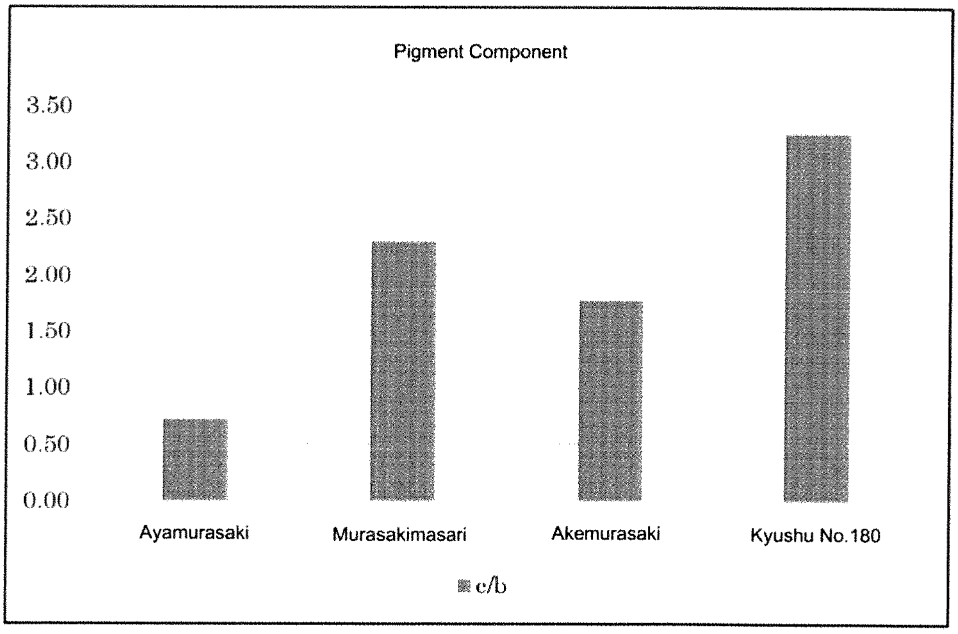

Fig. 7C

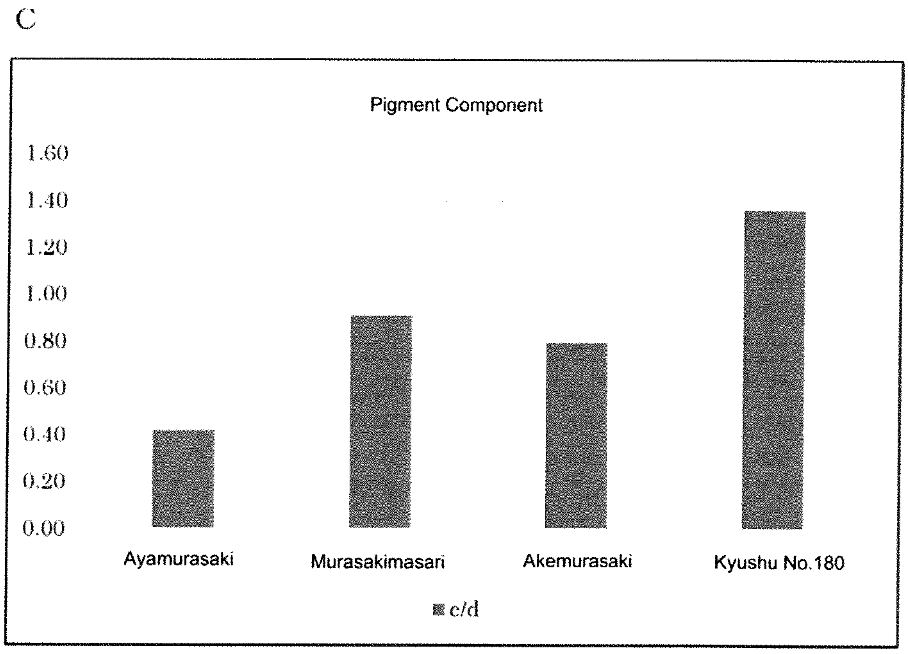
C
Pigment Component
1.60
1.40
1.20
1.00
0.80
0.60
0.40
0.20
0.00
Ayamurasaki
Murasakimasari
Akemurasaki
Kyushu No.180
c/d

Fig. 7D

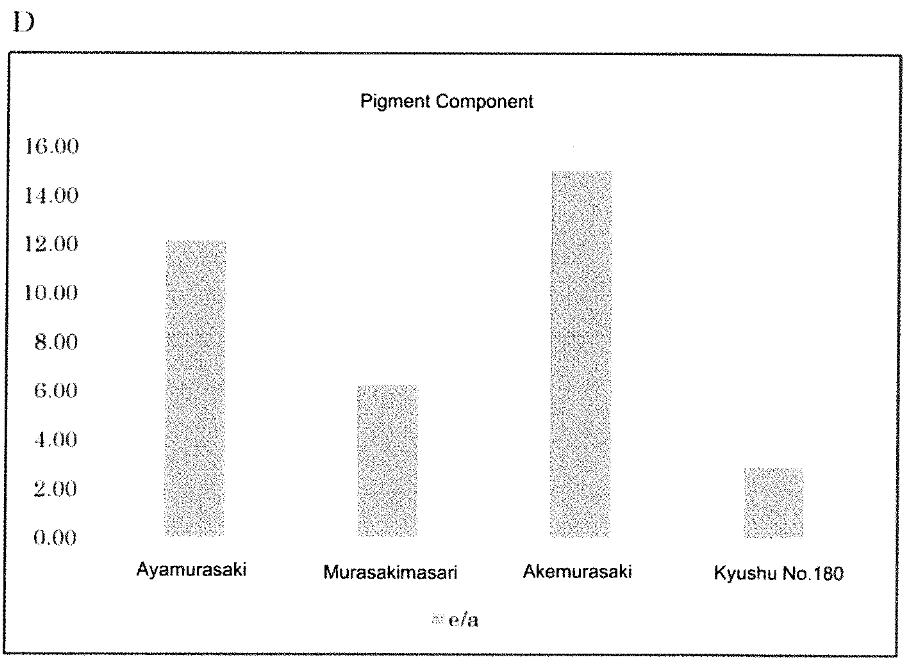
D
Pigment Component
16.00
14.00
12.00
10.00
8.00
6.00
4.00
2.00
0.00
Ayamurasaki
Murasakimasari
Akemurasaki
Kyushu No.180
e/a

Fig. 8

A

(2)Ultraviolet Component (Wavelength 320 nm)

| Peak Area | Aya masari | Murasaki masari | Ake masari | Kyushu No.180 |
|---|---|---|---|---|
| R.T. | YK1 | YK3 | YK4 | YK7 |
| 10.92 | 23676 | 30663 | 12308 | 46074 |
| 11.17 | | | 12980 | |
| 12.95 | 15653 | 13118 | | |
| 13.64 | | | | |
| 14.21 | 580106 | 865548 | 331593 | 902102 |
| 14.69 | 53436 | 41093 | 45809 | 34883 |
| 15.36 | 10066 | | | |
| 16.00 | | 15422 | 10609 | |
| 17.27 | 36981 | 24758 | 36296 | 21719 |
| 17.53 | | 11462 | | |
| 18.72 | 214598 | 261522 | 294025 | 249159 |
| 19.33 | 50974 | 72289 | 28123 | 53562 |
| 23.40 | 97505 | 74986 | 57168 | 61362 |
| 23.76 | 49600 | 53439 | 20701 | 53897 |
| 24.44 | 56736 | 84813 | 34357 | 117158 |
| 24.69 | 17419 | 11936 | 10277 | |
| Total Peak Area Detected at a Wavelength of 530 nm | 2756926 | 2578975 | 3114615 | 2619036 |

B

| Peak Area/Total Peak Area Detected at a Wavelength of 530 nm | Aya masari | Murasaki masari | Ake masari | Kyushu No.180 |
|---|---|---|---|---|
| R.T. | YK1 | YK3 | YK4 | YK7 |
| 10.92 | 0.0086 | 0.0119 | 0.0040 | 0.0176 |
| 11.17 | | | 0.0042 | |
| 12.95 | 0.0057 | 0.0051 | | |
| 13.64 | | | | |
| 14.21 | 0.2104 | 0.3356 | 0.1065 | 0.3444 |
| 14.69 | 0.0194 | 0.0159 | 0.0147 | 0.0133 |
| 15.36 | 0.0037 | | | |
| 16.00 | | 0.0060 | 0.0034 | |
| 17.27 | 0.0134 | 0.0096 | 0.0277 | 0.0083 |
| 17.53 | | 0.0044 | | |
| 18.72 | 0.0778 | 0.1014 | 0.0944 | 0.0951 |
| 19.33 | 0.0185 | 0.0280 | 0.0090 | 0.0205 |
| 23.40 | 0.0354 | 0.0291 | 0.0184 | 0.0234 |
| 23.76 | 0.0180 | 0.0207 | 0.0066 | 0.0206 |
| 24.44 | 0.0206 | 0.0329 | 0.0110 | 0.0447 |
| 24.69 | 0.0063 | 0.0046 | 0.0033 | |

Fig. 9

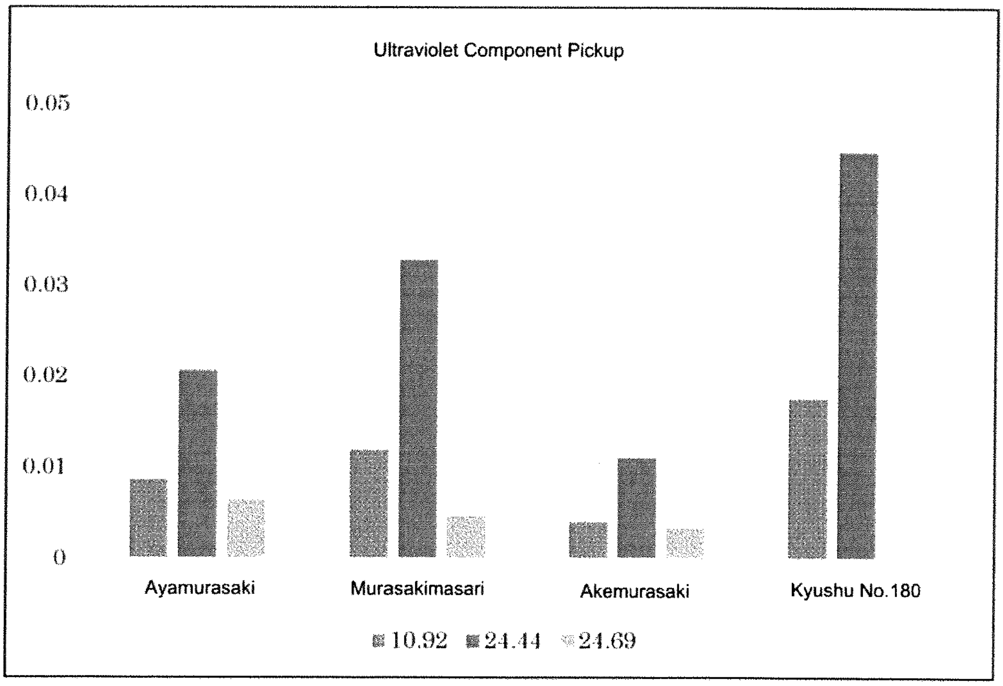

Fig. 10

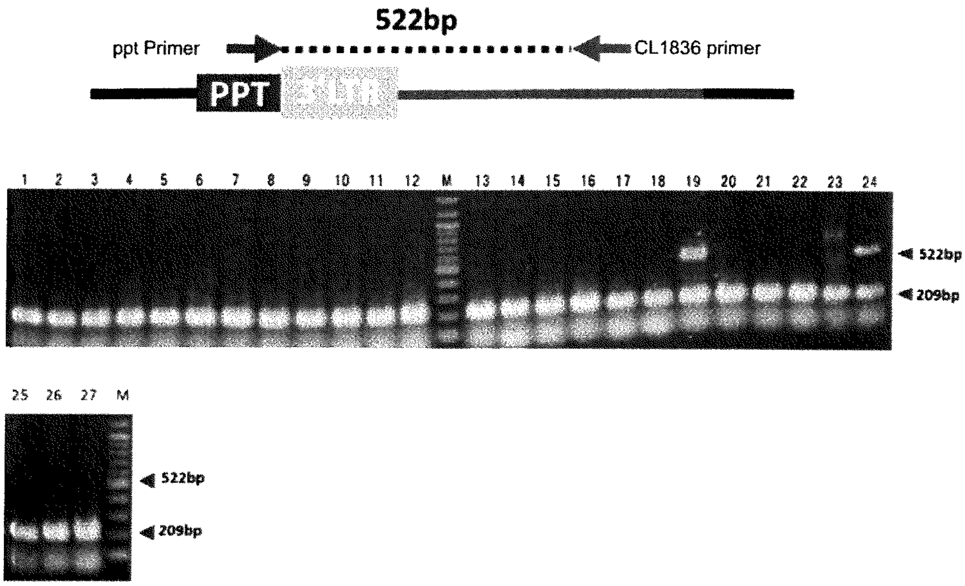


| | | | | | |
|---|---|---|---|---|---|
| 1 | Purple Sweet Road | 10 | *Satsumahikari* | 19 | *Kyushu* No. 180 |
| 2 | *Kyushu* No. 58 | 11 | *Kanto* No. 98 | 20 | *Kyukei* No. 316 |
| 3 | *Kuroshirazu* | 12 | *Kyukei* No. 174 | 21 | *Kyushu* No. 176 |
| 4 | *Tamayutaka* | 13 | *Shiroyutaka* | 22 | *Kyukei* No. 294 |
| 5 | *Shirosatsuma* | 14 | *Kyushu* No. 119 | 23 | A34-4 |
| 6 | *Kyukei* No. 165 | 15 | *Kanto* No. 99 | 24 | *Kyukei* 4208-2 |
| 7 | *Tanegashimamurasaki* | 16 | *Ayamurasaki* | 25 | *Kyukei* 4208-18 |
| 8 | *Kyushu* No. 137 | 17 | *Akemurasaki* | 26 | *Kyukei* 5282-26 |
| 9 | *Kyushu* No. 109 | 18 | *Murasakimasari* | 27 | *Kyukei* 4222-50 |

Fig. 11

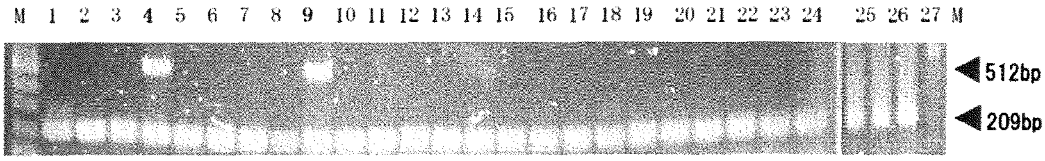


| | | | | | |
|---|---|---|---|---|---|
| 1 | *Ayamurasaki* | 10 | *Kyukei* 04208-18 | 19 | *Tanegashimamurasaki* |
| 2 | *Akemurasaki* | 11 | *Kyukei* 05282-26 | 20 | *Kyushu* No. 137 |
| 3 | *Murasakimasari* | 12 | *Kyukei* 04222-50 | 21 | *Kyushu* No. 109 |
| 4 | *Kyushu* No. 180 | 13 | Purple Sweet Road | 22 | *Satsumahikari* |
| 5 | *Kyukei* No. 316 | 14 | *Kyushu* No. 58 | 23 | *Kanto* No. 98 |
| 6 | *Kyushu* No. 176 | 15 | *Kuroshirazu* | 24 | *Kyukei* No. 174 |
| 7 | *Kyushu* No. 294 | 16 | *Tamayutaka* | 25 | *Shiroyutaka* |
| 8 | A34-4 | 17 | *Shirosatsuma* | 26 | *Kyushu* No. 119 |
| 9 | *Kyukei* 04208-2 | 18 | *Kyukei* No. 165 | 27 | *Kanto* No. 99 |

Fig. 12

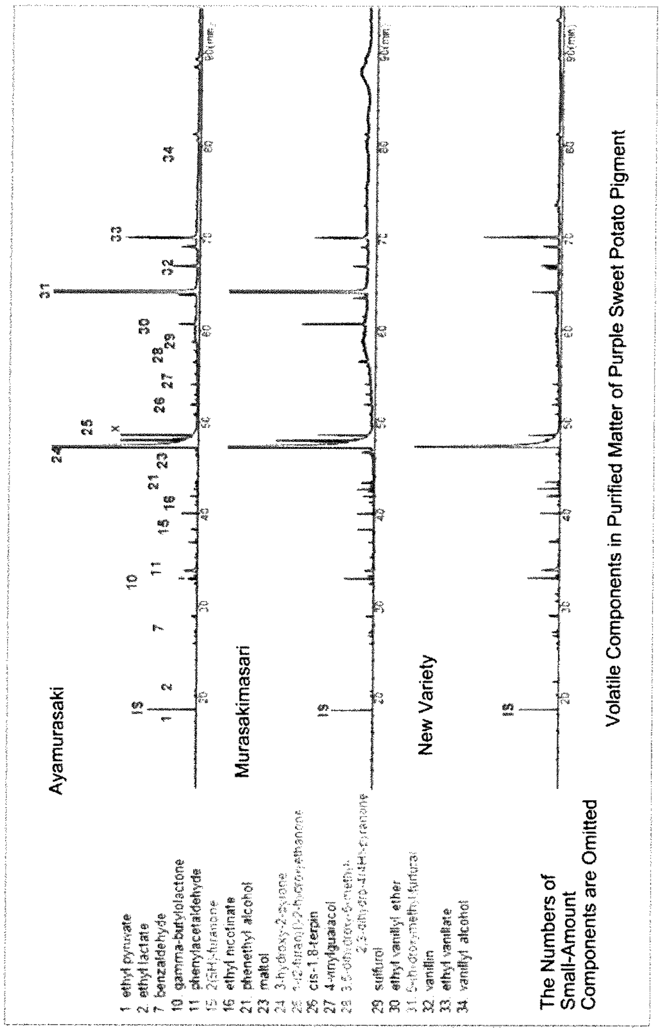
Ayamurasaki
Murasakimasari
New Variety
1 ethyl pyruvate
2 ethyl lactate
7 benzaldehyde
10 gamma-butyrolactone
11 phenylacetaldehyde
16 ethyl nicotinate
21 phenethyl alcohol
23 maltol
26 cis-1,8-terpin
27 4-vinylguaiacol
29 sulfurol
30 ethyl vanillyl ether
32 vanillin
33 ethyl vanillate
34 vanillyl alcohol
The Numbers of Small-Amount Components are Omitted
Volatile Components in Purified Matter of Purple Sweet Potato Pigment

Fig. 13

A

(1) Pigment Component (Wavelength 320 nm)

| Peak Area | Aya masari | Ake masari | Kyushu No.180 | Pigment Component |
|---|---|---|---|---|
| R.T. | YK1 | YK3 | YK7 | |
| 15.61 | 10822 | 24768 | 32759 | |
| 17.52 | 25642 | 44073 | 108126 | YGM-0e |
| 20.61 | 217110 | 433904 | 139334 | YGM-2 |
| 20.92 | 145787 | 231146 | 147180 | YGM-1a + YGM-1b |
| 21.69 | 693289 | 783244 | 707311 | YGM-3 + YGM-5b |
| 22.07 | 281182 | 158240 | 180343 | YGM-4b |
| 22.23 | 221366 | 286919 | 575676 | YGM-5a |
| 22.84 | 352429 | 251639 | 294286 | YGM-6 |
| Total Peak Area | 2262466 | 2656887 | 2498713 | |

B

| Peak Area/Total Peak Area Detected at a Wavelength of 530 nm | Aya masari | Ake masari | Kyushu No.180 |
|---|---|---|---|
| R.T. | YK1 | YK3 | YK7 |
| 15.61 | 0% | 1% | 1% |
| 17.52 | 1% | 2% | 4% |
| 20.61 | 10% | 16% | 6% |
| 20.92 | 6% | 9% | 6% |
| 21.69 | 31% | 29% | 28% |
| 22.07 | 12% | 6% | 7% |
| 22.23 | 10% | 11% | 23% |
| 22.84 | 16% | 8% | 12% |

Fig. 14

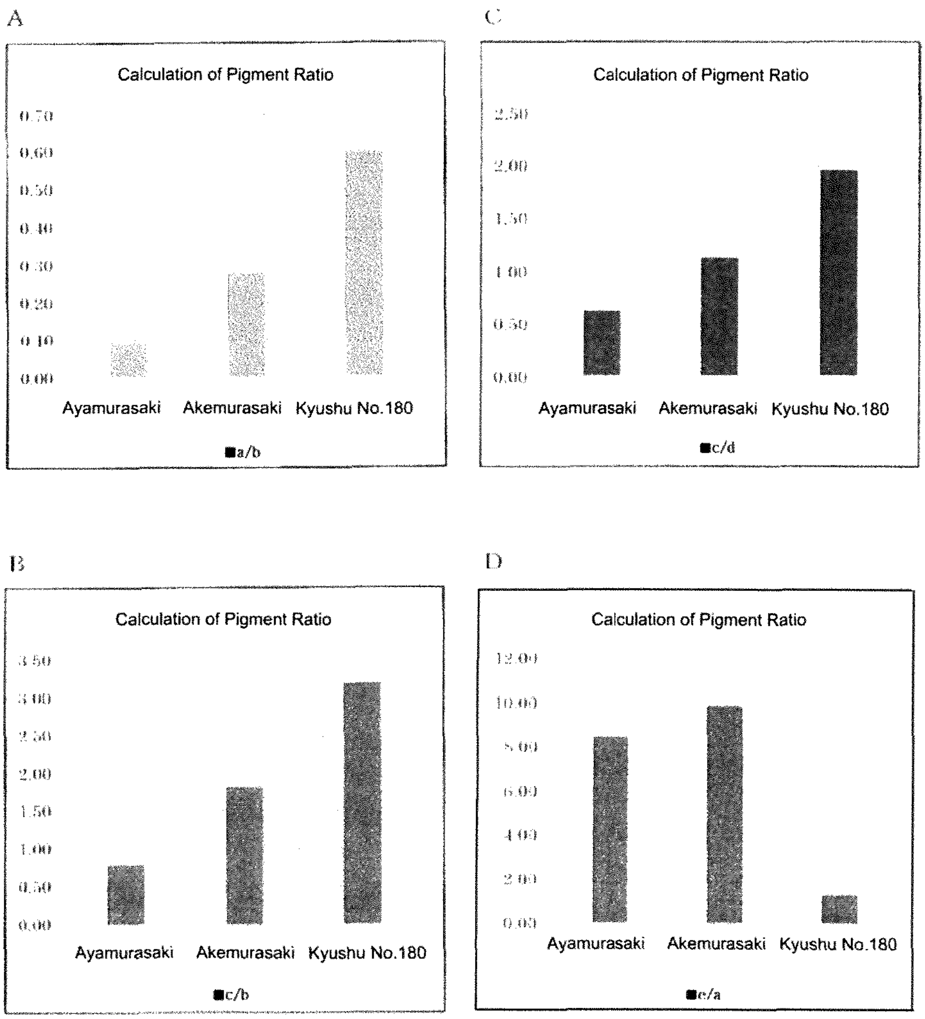

Fig. 15

A

(2)Ultraviolet Component (Wavelength 320 nm)

| Peak Area | Aya masari | Ake masari | Kyushu No.180 |
|---|---|---|---|
| R.T | YK1 | YK3 | YK7 |
| 10.92 | 38290 | 14571 | 54416 |
| 24.44 | | 12800 | 57215 |
| 24.69 | | | 11131 |
| Total Peak Area Detected at a Wavelength of 530 nm | 2262466 | 2656887 | 2498713 |

B

| Peak Area/Total Peak Area Detected at a Wavelength of 530 nm | Aya masari | Ake masari | Kyushu No.180 |
|---|---|---|---|
| R.T | YK1 | YK3 | YK7 |
| 10.92 | 0.0174 | 0.0055 | 0.0218 |
| 24.44 | 0.0000 | 0.0048 | 0.0229 |
| 24.69 | 0.0000 | 0.0000 | 0.0045 |

Fig. 16

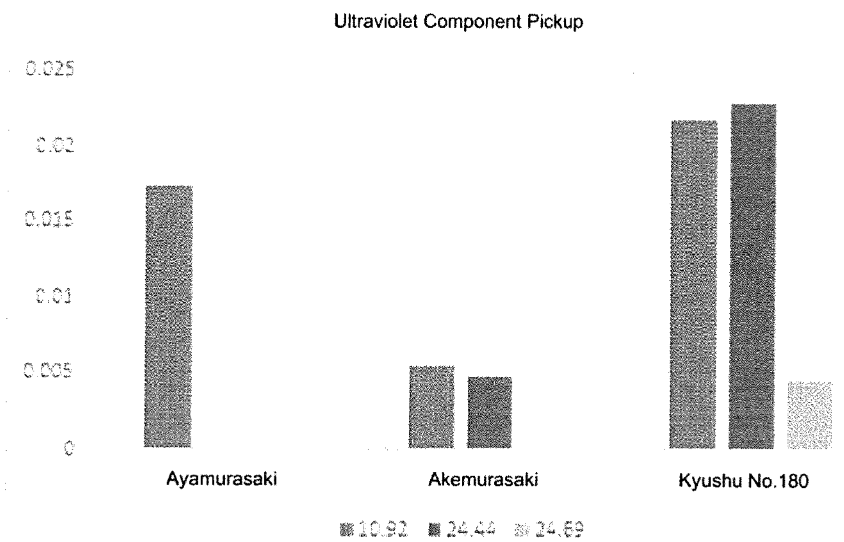

Fig. 17

A

(1) Pigment Component (Wavelength 530 nm)

| Peak Area | Kyushu No.180 | |
|---|---|---|
| R.T. | YK7 | |
| 15.61 | 234822 | |
| 17.52 | 506745 | YGM-0e |
| 20.61 | 986599 | YGM-2 |
| 20.92 | 756354 | YGM-1a+YGM-1b |
| 21.69 | 5578801 | YGM-3+YGM-5b |
| 22.07 | 721293 | YGM-4b |
| 22.23 | 2498556 | YGM-5a |
| 22.84 | 2035945 | YGM-6 |

Total Peak Area 15190078

B

| Peak Area/Total Peak Area Detected at a Wavelength of 530 nm | Kyushu No.180 |
|---|---|
| R.T. | YK7 |
| 15.61 | 2% |
| 17.52 | 3% |
| 20.61 | 6% |
| 20.92 | 5% |
| 21.69 | 37% |
| 22.07 | 5% |
| 22.23 | 16% |
| 22.84 | 13% |

Fig. 18

A

(1) Pigment Component (Wavelength 530 nm)

| Peak Area | Kyushu No.180 | |
|---|---|---|
| R.T. | YK7 | |
| 15.61 | 218183 | |
| 17.52 | 617580 | YGM-0e |
| 20.61 | 660300 | YGM-2 |
| 20.92 | 744806 | YGM-1a+YGM-1b |
| 21.69 | 3558373 | YGM-3+YGM-5b |
| 22.07 | 579518 | YGM-4b |
| 22.23 | 3179972 | YGM-5a |
| 22.84 | 1832595 | YGM-6 |

Total Peak Area 13586645

B

| Peak Area/Total Peak Area Detected at a Wavelength of 530 nm | Kyushu No.180 |
|---|---|
| R.T. | YK7 |
| 15.61 | 2% |
| 17.52 | 5% |
| 20.61 | 5% |
| 20.92 | 5% |
| 21.69 | 26% |
| 22.07 | 4% |
| 22.23 | 23% |
| 22.84 | 13% |

Fig. 19A

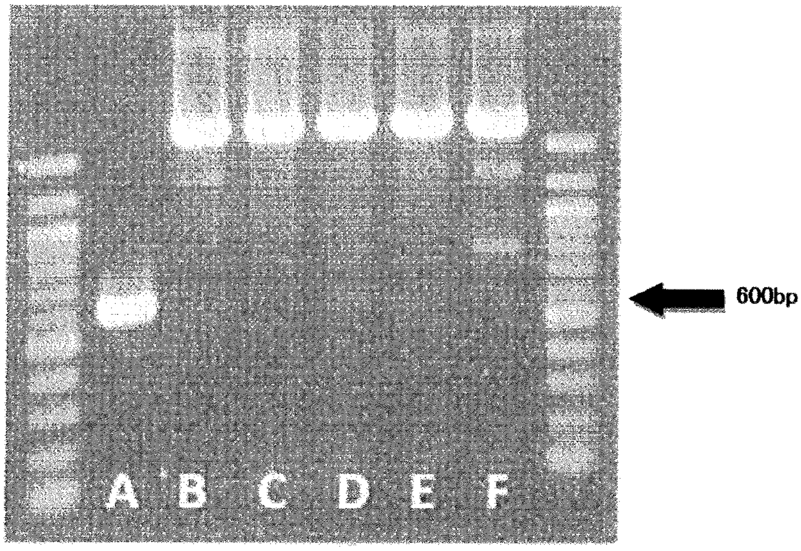


Fig. 19B

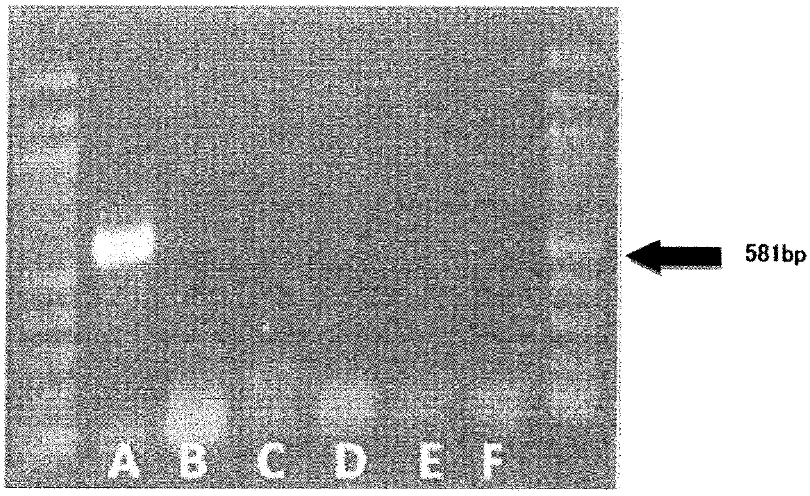

Fig. 19C

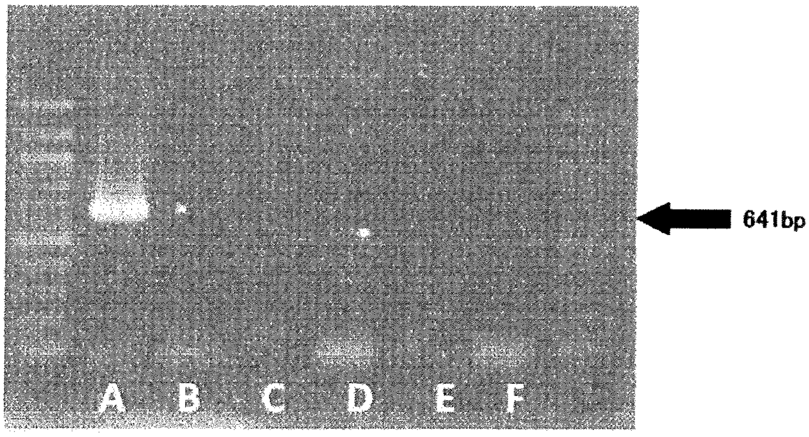


Fig. 19D

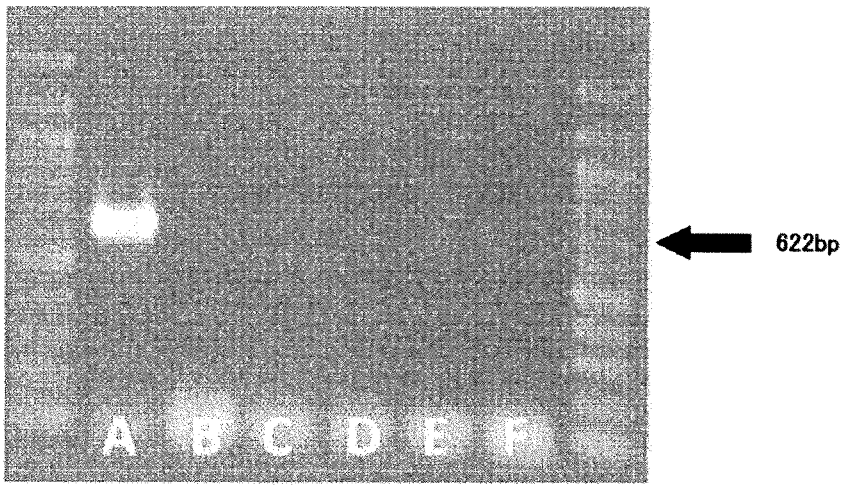

Fig. 19E

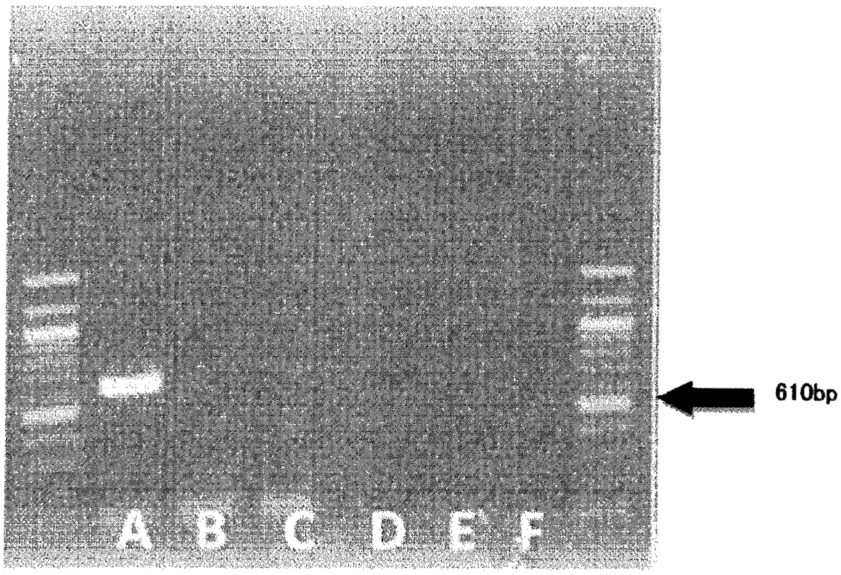


Fig. 19F

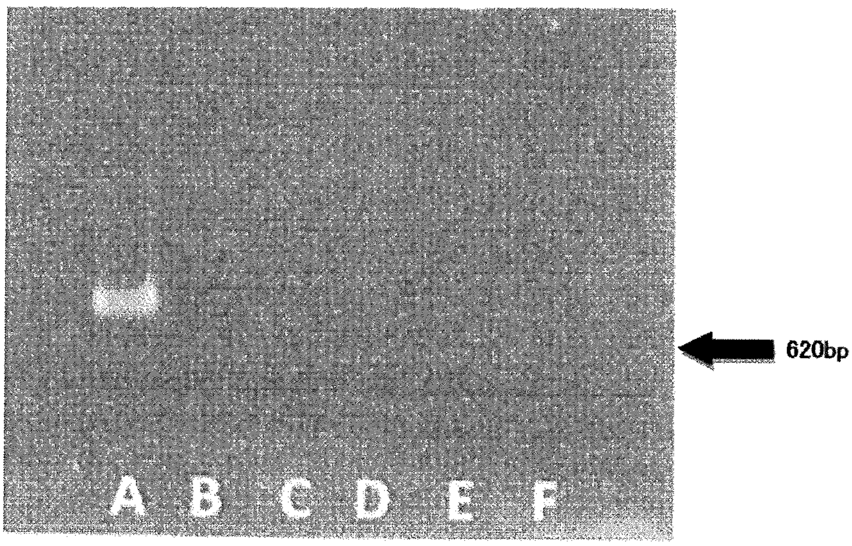

Fig. 19G

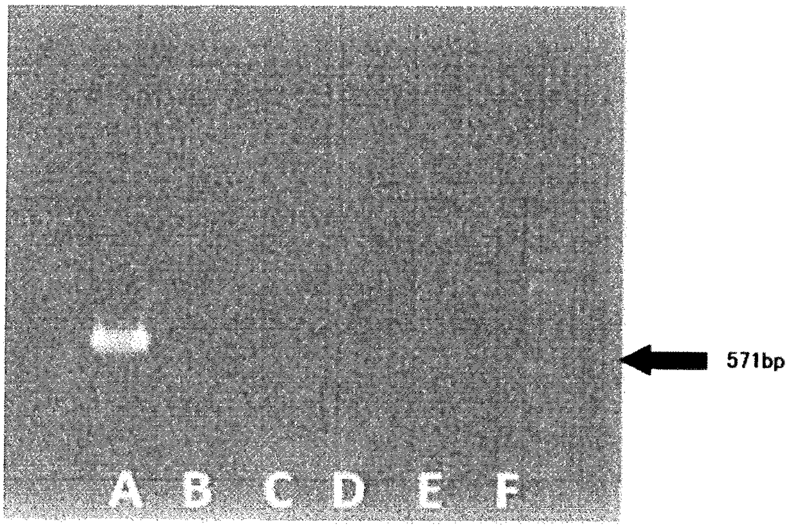

## INTERNATIONAL SEARCH REPORT

International application No.
PCT/JP2017/036028

A. CLASSIFICATION OF SUBJECT MATTER
Int.Cl. A01H5/06(2006.01)i, A23L5/43(2016.01)i, C12N15/09(2006.01)i, C12Q1/68(2006.01)i, A01H1/02(2006.01)n

According to International Patent Classification (IPC) or to both national classification and IPC

B. FIELDS SEARCHED

Minimum documentation searched (classification system followed by classification symbols)
Int.Cl. A01H1/00-5/12, A23L5/43, C12N15/00-15/90, C12Q1/68

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model specifications of Japan | 1994-2017 |

Electronic data base consulted during the international search (name of data base and, where practicable, search terms used)
DDBJ/GeneSeq, CAplus/MEDLINE/EMBASE/BIOSIS/WPIDS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>-<br>A | 境哲文外, サツマイモ新品種「アケムラサキ」の育成. 九州沖縄農業研究センター報告, 2010, vol. 53, pp. 1-23 (fugures, tables), (SAKAI, Tetsufumi, et al., "Akemurasaki": a new sweetpotato cultivar, Bulletin of the National Agricultural Research Center for Kyushu Okinawa Region) | 6-7<br>-<br>1-5, 8-15 |
| X<br>-<br>A | TRUONG V. D., et al., Characterization of anthocyanins and anthocyanidins in purple-fleshed sweetpotatoes by HPLC-DAD/ESI-MS/MS. J. Agric. Food. Chem., 2010, vol. 58, no. 1, pp. 404-410 (figures, tables) | 6-7<br>-<br>1-5, 8-15 |
| A | MONDEN Y., et al., Construction of a linkage map based on retrotransposon insertion polymorphisms in sweetpotato via high-throughput sequencing. Breed. Sci., 2015, vol. 65, no. 2, pp. 145-153 | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 November 2017 (27.11.2017) | 12 December 2017 (12.12.2017) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- NARO List of Providers of Varieties Grown. Kyushu Okinawa Agricultural Research Center, 18 April 2016
- **TAHARA, M. et al.** *Mol Gen. Genomics,* 2004, vol. 272, 116-127
- **MONDEN, Y. et al.** *DNA Res.,* 2014, vol. 21, 491-498
- Japanese Standards of Food Additives. Japan Ministry of Health, Labour and Welfare
- **TAKAHIKO HAYAKAWA.** New Edition of Plant PCR Experiment Protocol. Shujunsha, 1997, 49-56
- Manual of Assessment and Analysis for Genetically Modified Foods. JAS Analytical Test Handbook. 2002
- **TAKAHIKO HAYAKAWA.** *New Edition of Plant PCR Experiment Protocol,* 1997, 49-56
- **NATSUKO OOE et al.** Determination of Variety of Material of Sweet Potato Processed Product using Retrotransposon. *Breeding Research,* 2004, vol. 6, 169-177
- **TOSHIKAZU KURANOUCH et al.** Study of Quality Improvement of Steamed and Dried Sweet Potato Slices. *Journal of Crop Research (Bull. Natl. Inst. Crop Sci.),* March 2010, vol. 11, 49-65